# EUROPEAN PATENT APPLICATION

(11) **EP 1 897 880 A1**
(43) Date of publication of application: **12.03.2008**
(21) Application number: 06747302.5
(22) Date of filing: 08.06.2006
(51) Int. Cl.: C07D 417/04, A61K 31/5415, A61P 3/10, A61P 9/00, A61P 13/12, A61P 19/00, A61P 25/00, A61P 29/00, A61P 31/00, A61P 35/00, A61P 39/06, A61P 43/00, C07D 417/14

(54) **1,3-BENZOTHIAZINONE DERIVATIVE AND USE THEREOF**

(30) Priority: 09.06.2005 JP 2005170054
(71) Applicant: Takeda Pharmaceutical Company Limited, Osaka-shi, Osaka 541-0045 (JP)
(72) Inventor: KAJINO, Masahiro, c/o TAKEDA PHARMAC. CO. LTD., Osaka-shi, Osaka 532-8686 (JP); IMAEDA, Toshihiro, c/o TAKEDA PHARM. COMPANY LTD., Osaka-shi, Osaka 532-8686 (JP)
(74) Representative: Rickard, Timothy Mark Adrian
(86) International application number: PCT/JP2006/311977
(87) International publication number: WO 2006/132438

(57) **Abstract**

The present invention provides compounds represented by formula (I): 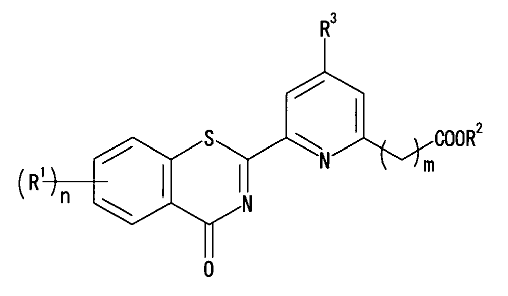 wherein R¹ independently represents a halogen atom, hydroxy, nitro, an optionally halogenated alkyl, alkenyl or alkynyl, an optionally substituted alkoxy, an acyl, or an optionally substituted amino; R² represents hydrogen atom or an optionally substituted hydrocarbon group; R³ represents an electron-withdrawing group; n represents an integer of 0 to 2; and m represents an integer of 1 to 6 (hereinafter sometimes briefly referred to as Compound (I)); or salts thereof. These compounds are capable of binding to macrophage migration inhibitory factor (MIF) and useful as agents for preventing/treating diseases induced by oxidative stress.

SEQUENCE LISTING

<110>  Takeda Pharmaceutical Company Limited

<120>  1,3-BENZOTHIAZINONE DERIVATIVES AND USE THEREOF

<130>  G07-0126US

<150>  JP2005-170054
<151>  2005-06-09

<160>  4

<170>  PatentIn version 3.3

<210>  1
<211>  42
<212>  DNA
<213>  Rattus norvegicus

<400>  1
gagcttggaa atggcattgc taatggtgac aaagcaactt tg                    42


<210>  2
<211>  13
<212>  DNA
<213>  Homo sapiens

<400>  2
aggacaaagg tca                                                    13


<210>  3
<211>  27
<212>  DNA
<213>  Homo sapiens

<400>  3
ccatatgccg atgttcatcg taaacac                                     27


<210>  4
<211>  39
<212>  DNA
<213>  Homo sapiens

<400>  4
gaagaagctc ttccgcaggc gaaggtggag ttgttccag                        39

## Description

### TECHNICAL FIELD

The present invention relates to novel 1,3-benzothiazinone derivatives.

### BACKGROUND ART

The reactive oxygen species generated as by-products of cell respiration or metabolism oxidizes DNAs, lipids, proteins, etc. to induce damages in cells or tissues. For this reason, a variety of defense systems normally work to protect cells or tissues from such oxidative stress. However, when a balance is disrupted between the protection and production systems for some reasons, the living body will lapse into the undesirable conditions. Actually, it is pointed out that oxidative stress plays a role in the onset and aggravation of various diseases including circulatory disorders, bone/joint disorders, infectious disorders, inflammatory disorders, renal disorders, central nervous disorders, cancer, diabetes mellitus, and the like (Current Pharmaceutical Design, 10, 879, 2004; Alimentary Pharmacology and Therapeutics, 20, 177, 2004; Journal of Investigative Medicine, 49, 566, 2001).

The antioxidant response element (ARE) regulates the expression of diverse protective factors against oxidative stress and its transcriptional activation is considered as one of the most important cytoprotective mechanisms against oxidative stress (Current Pharmaceutical Design, 10, 879, 2004). It is also pointed out the possibility that the expression and increased levels ofARE-regulated protective factors would play a crucial role in pharmaceutical effects of gold preparations and NSAIDs used for the treatment of rheumatoid (The Journal of Biological Chemistry, 276, 34074, 2001; Free Radical Biology and Medicine, 37, 650, 2002). Based on these findings, it is assumed that ARE activation will result in prevention of disorders associated with oxidative stress. In fact, a trend toward the development of ARE activators as therapeutic agents based on this assumption is going on forward but any clinically available compound is not yet known (Kagaku-To-Seibutsu (Bioscience, Biotechnology, and Agrochemistry), 43, 20, 2005). On the other hand, it is reported in WO 03/090782 and JPA 2004-196792 that low molecular compounds or 1,3-benzothiazinone derivatives, which bind to the macrophage migration inhibitory factor (MIF), upregulate the expression of genes under control by ARE to exhibit cytoprotective effects.

### DISCLOSURE OF INVENTION

It has been earnestly desired to develop pharmaceuticals such as safe MIF-binding agents, antioxidant response element (hereinafter sometimes referred to as "ARE") activators, cytoprotective agents, etc., having excellent pharmacokinetics and minimized side effects.

In view of the foregoing circumstances, the present inventors have made extensive studies and found that Compound (I) is capable of binding to MIF, possesses excellent cytoprotective and cell death suppression activities, has the activities of increasing the expression of genes (e.g., genes for factors protecting cells from various stresses, etc.) under control of ARE, the activities of upregulating (promoting) the production or activation of gene proteins (gene products) under control of ARE, etc. and that Compound (I) is remarkably excellent in pharmacokinetics (oral absorption, etc.) and has markedly reduced side effects. Based on these findings, the present inventors have made further investigations and come to accomplish the present invention.

In other words, the present invention relates to the following features.
(1) A compound represented by formula (I): 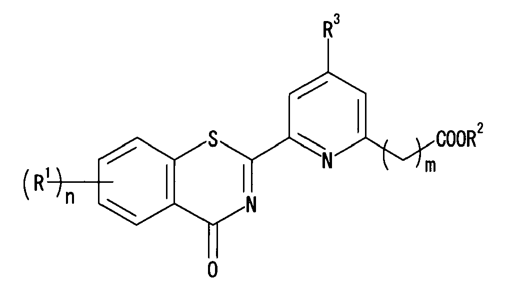 wherein R¹ independently represents a halogen atom, hydroxy, nitro, an optionally halogenated alkyl, alkenyl or alkynyl, an optionally substituted alkoxy, an acyl, or an optionally substituted amino; R² represents hydrogen atom or an optionally substituted hydrocarbon group; R³ represents an electron-withdrawing group; n represents an integer of 0 to 2; and m represents an integer of 1 to 6 (hereinafter sometimes briefly referred to as Compound (I)); or a salt thereof.
(2) The compound according to (1) above, or a salt thereof, wherein R¹ is a halogen atom.
(3) The compound according to (1) above, or a salt thereof, wherein R² is hydrogen atom or a C₁₋₆ alkyl group.
(4) The compound according to (1) above, or a salt thereof, wherein the electron-withdrawing group is a halogen atom, cyano group, nitro group, a halogenomethyl group, a group represented by formula -S(O)ₚ-R⁴ (wherein p represents 1 or 2 and R⁴ represents hydrogen atom or an optionally substituted hydrocarbon group), a group represented by formula -COOR⁵ (R⁵ represents hydrogen atom or an optionally substituted hydrocarbon group), a group represented by formula -CONR⁶R⁷ (wherein R⁶ and R⁷ may be the same or different and independently represent hydrogen atom or an optionally substituted hydrocarbon group), a group represented by formula -SO₂NH-R⁸ (wherein R⁸ represents hydrogen atom or an optionally substituted hydrocarbon group), or a group represented by formula -CO-R⁹ (wherein R⁹ represents hydrogen atom or an optionally substituted hydrocarbon group).
(5) The compound according to (1) above, or a salt thereof, wherein said compound or a salt thereof is selected from the group consisting of
   3-[6-(4-oxo-4H-1,3-benzothiazin-2-yl)-4-(methylsulfonyl)pyridin-2-yl]propanoic acid;
   3-[6-(7-chloro-4-oxo-4H-1,3-benzothiazin-2-yl)-4-(methylsulfonyl)pyridin-2-yl]prop anoic acid,
   3-[6-(7-fluoro-4-oxo-4H-1,3-benzothiazin-2-yl)-4-(methylsulfonyl)pyridin-2-yl]prop anoic acid;
   3-[6-(7-methyl-4-oxo-4H-1,3-benzothiazin-2-yl)-4-(methylsulfonyl)pyridin-2-yl]pro panoic acid; 2-(diethylamino)ethyl
   3-[6-(7-chloro-4-oxo-4H-1,3-benzothiazin-2-yl)-4-(methylsulfonyl)pyridin-2-yl]prop anoate; and, 2-(diethylamino)-2-oxoethyl
   3-[6-(7-chloro-4-oxo-4H-1,3-benzothiazin-2-yl)-4-(methylsulfonyl)pyridin-2-yl]prop anoate.
(6) A macrophage migration inhibitory factor (MIF)-binding agent comprising the compound according to (1) above, or a salt thereof, or a prodrug thereof.
(7) An antioxidant response element activator comprising the compound according to (1) above, or a salt thereof, or a prodrug thereof.
(8) A cytoprotective agent comprising the compound according to (1) above, or a salt thereof, or a prodrug thereof.
(9) A medicament comprising the compound according to (1) above, or a salt thereof, or a prodrug thereof.
(10) The medicament according to (9) above, which is an agent for preventing/treating disorders caused by oxidative stress.
(11) The medicament according to (9) above, which is an agent for preventing/treating circulatory disorders, bone/joint disorders, infectious disorders, inflammatory disorders, renal disorders, central nervous disorders, cancer or diabetes mellitus.
(12) A method for preventing/treating circulatory disorders, bone/joint disorders, infectious disorders, inflammatory disorders, renal disorders, central nervous disorders, cancer or diabetes mellitus, which comprises administering to a mammal an effective dose of the compound according to (1) above, or a salt thereof, or a prodrug thereof.
(13) Use of the compound according to (1) above, or a salt thereof, or a prodrug thereof, to manufacture an agent for preventing/treating circulatory disorders, bone/joint disorders, infectious disorders, inflammatory disorders, renal disorders, central nervous disorders, cancer or diabetes mellitus.

### BRIEF DESCRIPTION OF THE DRAWING

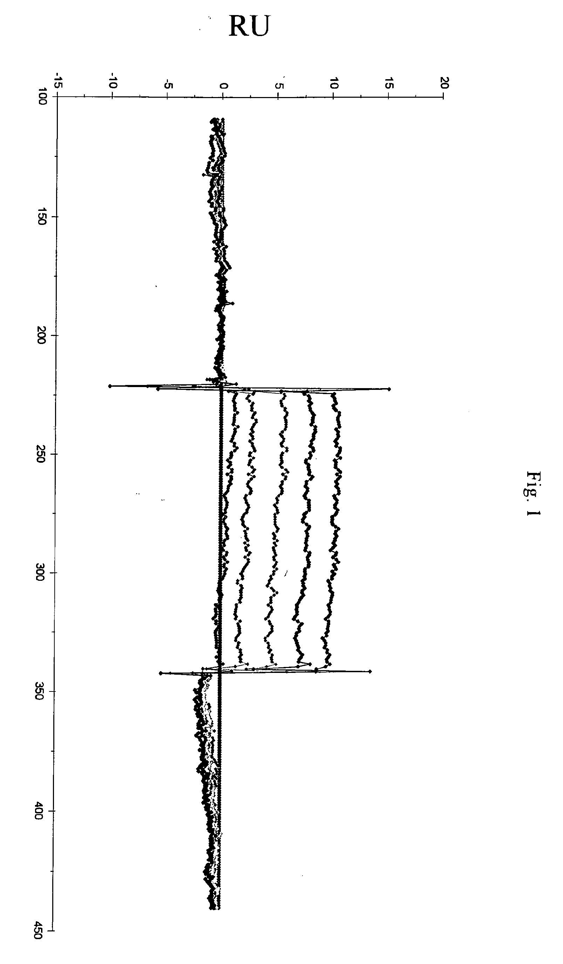

FIG 1 shows the results of binding MIF to the compound of EXAMPLE 8.

### BEST MODE FOR CARRYING OUT THE INVENTION

Hereinafter, the present invention is described in detail. The following embodiments are presented by way of example to explain the present invention but are not deemed to limit the present invention to these embodiments only. The present invention may be embodied in various modifications unless it is departed from the gist of the invention.

An embodiment of the present invention is directed to the compounds represented by formula (I) above [wherein R¹ independently represents a halogen atom, hydroxy, nitro, an optionally halogenated alkyl, alkenyl or alkynyl, an optionally substituted alkoxy, an acyl, or an optionally substituted amino; R² represents hydrogen atom or an optionally substituted hydrocarbon group; R³ represents an electron-withdrawing group; n represents an integer of 0 to 2; and m represents an integer of 1 to 6]; or salts thereof.

In the formula (I) described above, R¹ shows a substituent on the benzene ring of the 1,3-benzothiazinone skeleton and represents a halogen atom, hydroxy, nitro, an optionally halogenated alkyl, alkenyl or alkynyl, an optionally substituted alkoxy, an acyl, or an optionally substituted amino. Preferably, R¹ is a halogen atom, hydroxy, nitro, an optionally halogenated alkyl, an optionally substituted alkoxy, an acyl, or an optionally substituted amino.

The "halogen atom" shown by R¹ includes, for example, fluorine, chlorine, bromine, iodine, etc.

The "optionally halogenated alkyl" represented by R¹ includes, for example, an alkyl (e.g., C₁₋₆ alkyl such as methyl, ethyl, propyl, isopropyl, butyl, isobutyl, sec-butyl, tert-butyl, pentyl, hexyl, etc.) optionally having 1 to 5, preferably 1 to 3 halogen atoms (e.g., fluorine, chlorine, bromine, iodine, etc.), etc. Specific examples are methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc.

The "optionally halogenated alkenyl or alkynyl" represented by R¹ includes, for example, those containing 1 or 2 double bonds or triple bonds in the "optionally halogenated alkyl" described above.

The "alkoxy" in the "optionally substituted alkoxy" represented by R¹ includes, for example, C₁₋₈alkoxy such as methoxy, ethoxy, propoxy, isopropoxy, butoxy, isobutoxy, sec-butoxy, pentyloxy, hexyloxy, etc.

The "substituent" in the "optionally substituted alkoxy" represented by R¹ includes the same substituents exemplified as "substituents" given for the "optionally substituted hydrocarbon group" represented by R¹⁰, R¹¹, R¹², R¹⁴, R¹⁵, R¹⁶ or R¹⁷ later described, which substituents may be 1 to 3 at substitutable positions.

The "acyl" represented by R¹ includes, for example, an acyl represented by formula: -(C=O)-R¹⁰, (C=O)-OR¹¹, -(C=O)-NR¹²R¹³, -(C=S)-NHR¹⁴, -SO-R¹⁵, -SO₂-R¹⁶ or -SO₂-NHR¹⁷ [wherein each of R¹⁰, R¹¹, R¹², R¹⁴ and R¹⁷ independently represents hydrogen atom, an optionally substituted hydrocarbon group or an optionally substituted heterocyclic group; R¹³ represents hydrogen atom or C₁₋₆ alkyl; and R¹⁵ or R¹⁶ represent an optionally substituted hydrocarbon group or an optionally substituted heterocyclic group].

The "hydrocarbon group" in the "optionally substituted hydrocarbon group" represented by R¹⁰, R¹¹, R¹², R¹⁴, R¹⁵, R¹⁶ or R¹⁷ includes, for example, a linear or cyclic hydrocarbon group (e.g., an alkyl, alkenyl, alkynyl, cycloalkyl, aryl, aralkyl, etc.) and the like. Among them, preferred are a linear or cyclic hydrocarbon group having 1 to 16 carbon atoms, and the like.

The "alkyl" preferably includes, for example, C₁₋₆ alkyl (e.g., methyl, ethyl, propyl, isopropyl, butyl, isobutyl, sec-butyl, tert-butyl, pentyl, hexyl, etc.), and the like.

The "alkenyl" preferably includes, for example, C₂₋₆ alkenyl (e.g., vinyl, allyl, isopropenyl, 1-butenyl, 2-butenyl, 3-butenyl, 2-methyl-2-propenyl, 1-methyl-2-propenyl, 2-methyl-1-propenyl, etc.), and the like.

The "alkynyl" preferably includes, for example, C₂₋₆ alkynyl (e.g., ethynyl, propargyl, 1-butynyl, 2-butynyl, 3-butynyl, 1-hexynyl, etc.), and the like.

The "cycloalkyl" preferably includes, for example, C₃₋₆ cycloalkyl (e.g., cyclopropyl, cyclobutyl, cyclopentyl, cyclohexyl, etc.), and the like.

The "aryl" preferably includes, for example, C₆₋₁₄ aryl (e.g., phenyl, 1-naphthyl, 2-naphthyl, 2-biphenylyl, 3-biphenylyl,4-biphenylyl, 2-anthryl, etc.), and the like.

The "aralkyl" preferably includes, for example, C₇₋₆ aralkyl (e.g., benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, etc.), and the like.

In the "optionally substituted hydrocarbon group" represented by R¹⁰, R¹¹, R¹², R¹⁴, R¹⁵, R¹⁶ or R¹⁷, the "substituent" includes, for example, a halogen atom (e.g., fluorine, chlorine, bromine, iodine, etc.), C₁₋₃ alkylenedioxy (e.g., methylenedioxy, ethylenedioxy, etc.), nitro, cyano, optionally halogenated C₁₋₆ alkyl, optionally halogenated C₂₋₆ alkenyl, carboxy-C₂₋₆ alkenyl (e.g., 2-carboxyethenyl, 2-carboxy-2-methylethenyl, etc.), optionally halogenated C₂₋₆ alkynyl, optionally halogenated C₃₋₆ cycloalkyl, C₆₋₁₄ aryl (e.g., phenyl, 1-naphthyl, 2-naphthyl, 2-biphenylyl, 3-biphenylyl, 4-biphenylyl, 2-anthryl, etc.), optionally halogenated C₁₋₈ alkoxy, C₁₋₆ alkoxy-carbonyl-C₁₋₆ alkoxy (e.g., ethoxycarbonyloxy, etc.), hydroxy, C₆₋₁₄ aryloxy (e.g., phenyloxy, 1-naphthyloxy, 2-naphthyloxy, etc.), C₇₋₁₆ aralkyloxy (e.g., benzyloxy, phenethyloxy, etc.), mercapto, optionally halogenated C₁₋₆ alkylthio, C₆₋₁₄ arylthio (e.g., phenylthio, 1-naphthylthio,2-naphthylthio, etc.), C₇₋₁₆ aralkylthio (e.g., benzylthio, phenethylthio, etc.), amino, mono-C₁₋₆ alkylamino (e.g., methylamino, ethylamino, etc.), mono-C₆₋₁₄ arylamino (e.g., phenylamino, 1-naphthylamino, 2-naphthylamino, etc.), di-C₁₋₆ alkylamino (e.g., dimethylamino, diethylamino, ethylmethylamino, etc.), di-C₆₋₁₄ arylamino (e.g., diphenylamino, etc.), formyl, carboxy, C₁₋₆ alkyl-carbonyl (e.g., acetyl, propionyl, etc.), C₃₋₆ cycloalkyl-carbonyl (e.g., cyclopropylcarbonyl, cyclopentylcarbonyl, cyclohexylcarbonyl, etc.), C₁₋₆ alkoxy-carbonyl (e.g., methoxycarbonyl, ethoxycarbonyl, propoxycarbonyl, tert-butoxycarbonyl, etc.), C₆₋₁₄ aryl-carbonyl (e.g., benzoyl, 1-naphthoyl, 2-naphthoyl, etc.), C₇₋₁₆ aralkyl-carbonyl (e.g., phenylacetyl, 3-phenylpropionyl, etc.), C₆₋₁₄ aryloxy-carbonyl (e.g., phenoxycarbonyl, etc.), C₇₋₁₆ aralkyloxy-carbonyl (e.g., benzyloxycarbonyl, phenethyloxycarbonyl, etc.), 5- or 6-membered heterocyclic carbonyl (e.g., nicotinoyl, isonicotinoyl, thenoyl, furoyl, morpholinocarbonyl, thiomorpholinocarbonyl, piperazin-1-ylcarbonyl,pyrrolidin-1-ylcarbonyl, etc.), carbamoyl, mono-C₁₋₆ alkyl-carbamoyl (e.g., methylcarbamoyl, ethylcarbamoyl, etc.), di-C₁₋₆ alkyl-carbamoyl (e.g., dimethylcarbamoyl, diethylcarbamoyl, ethylmethylcarbamoyl, etc.), C₆₋₁₄ aryl-carbamoyl (e.g., phenylcarbamoyl, 1-naphthylcarbamoyl, 2-naphthylcarbamoyl, etc.), 5- or 6-membered heterocyclic carbamoyl (e.g., 2-pyridylcarbamoyl, 3-pyridylcarbamoyl, 4-pyridylcarbamoyl, 2-thienylcarbamoyl, 3-thienylcarbamoyl, etc.), C₁₋₆ alkylsulfonyl (e.g., methylsulfonyl, ethylsulfonyl, etc.), C₆₋₁₄ arylsulfonyl (e.g., phenylsulfonyl, 1-naphthylsulfonyl, 2-naphthylsulfonyl, etc.), formylamino, C₁₋₆ alkyl-carbonylamino (e.g., acetylamino, etc.), C₆₋₁₄ aryl-carbonylamino (e.g., benzoylamino, naphthoylamino, etc.), C₁₋₆ alkoxy-carbonylamino (e.g., methoxycarbonylamino, ethoxycarbonylamino, propoxycarbonylamino, butoxycarbonylamino, etc.), C₁₋₆ alkylsulfonylamino (e.g., methylsulfonylamino, ethylsulfonylamino, etc.), C₆₋₁₄ arylsulfonylamino (e.g., phenylsulfonylamino, 2-naphthylsulfonylamino, 1-naphthylsulfonylamino, etc.), C₁₋₆ alkyl-carbonyloxy (e.g., acetoxy, propionyloxy, etc.), C₆₋₁₄ aryl-carbonyloxy (e.g., benzoyloxy, naphthylcarbonyloxy, etc.), C₁₋₆ alkoxy-carbonyloxy(e.g., methoxycarbonyloxy, ethoxycarbonyloxy, propoxycarbonyloxy, butoxycarbonyloxy, etc.), mono- C₁₋₆ alkyl-carbamoyloxy (e.g., methylcarbamoyloxy, ethylcarbamoyloxy, etc.),di-C₁₋₆ alkyl-carbamoyloxy(e.g., dimethylcarbamoyloxy, diethylcarbamoyloxy, etc.), C₆₋₁₄ aryl-carbamoyloxy(e.g., phenylcarbamoyloxy, naphthylcarbamoyloxy, etc.), nicotinoyloxy, optionally substituted 5- to 7-membered saturated cyclic amino, 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.), sulfo, 2-oxo-1,3-dioxolen-4-yl which may be optionally substituted with C₁₋₆ alkyl (e.g., methyl, ethyl, propyl, isopropyl, butyl, isobutyl, sec-butyl, tert-butyl, pentyl, hexyl, etc.), and the like.

The "hydrocarbon group" may have, for example, 1 to 5, preferably 1 to 3, of the above substituents at substitutable positions. When the number of substituents is 2 or more, the respective substituents may be the same or different.

The "optionally halogenated C₁₋₆ alkyl" described above includes alkyl (e.g., C₁₋₆ alkyl such as methyl, ethyl, propyl, isopropyl, butyl, isobutyl, sec-butyl, tert-butyl, pentyl, hexyl, etc.) which may optionally have, for example, 1 to 5, preferably 1 to 3 halogen atoms (e.g., fluorine, chlorine, bromine, iodine, etc.), and the like. Specific examples include methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl,isobutyl, sec-butyl, tert-butyl, pentyl, isopentyl, neopentyl, 5,5,5-trifluoropentyl, hexyl, 6,6,6-trifluorohexyl, and the like.

The "optionally halogenated C₂₋₆ alkenyl" described above includes, for example, C₂₋₆ alkenyl (e.g., vinyl, propenyl, isopropenyl, 2-buten-1-yl, 4-penten-1-yl, S-hexen-1-yl, etc.) which may have, e.g., 1 to 5, preferably 1 to 3 halogen atoms (e.g., fluorine, chlorine, bromine, iodine, etc.), and the like.

The "optionally halogenated C₂₋₆ alkynyl" described above includes, for example, C₂₋₆ alkynyl (e.g., 2-butyn-1-yl, 4-pentyn-1-yl, 5-hexyn-1-yl, etc.), which may have, e.g., 1 to 5, preferably 1 to 3 halogen atoms (e.g., fluorine, chlorine, bromine, iodine, etc.), and the like.

The "optionally halogenated C₃₋₆ cycloalkyl" described above includes C₃₋₆ cycloalkyl (e.g., cyclopropyl, cyclobutyl, cyclopentyl, cyclohexyl, etc.), which may have, e.g., 1 to 5, preferably 1 to 3 halogen atoms (e.g., fluorine, chlorine, bromine, iodine, etc.), and the like. Specific examples are cyclopropyl, cyclobutyl, cyclopentyl, cyclohexyl, 4,4-dichlorocyclohexyl, 2,2,3,3-tetrafluorocyclopentyl, 4-chlorocyclohexyl, etc.

The "optionally halogenated C₁₋₈ alkoxy" described above includes C₁₋₈ alkoxy (e.g., cyclopropyl, cyclobutyl, cyclopentyl, cyclohexyl, etc.), which may have, e.g., 1 to 5, preferably 1 to 3 halogen atoms (e.g., fluorine, chlorine, bromine, iodine, etc.), and the like. Specific examples are methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, etc.

The "optionally halogenated C₁₋₆ alkylthio" described above includes C₁₋₆ alkylthio (e.g., methylthio, ethylthio, propylthio, isopropylthio, butylthio, sec-butylthio, tert-butylthio, etc.), which may have, e.g., 1 to 5, preferably 1 to 3 halogen atoms (e.g., fluorine, chlorine, bromine, iodine, etc.), and the like. Specific examples are methylthio, difluoromethylthio, trifluoromethylthio, ethylthio, propylthio, isopropylthio, butylthio, 4,4,4-trifluorobutylthio, pentylthio, hexylthio, etc.

In the "optionally substituted 5- to 7-membered saturated cyclic amino" described above, the "5- to 7-membered saturated cyclic amino" includes, for example, 5- to 7-membered saturated cyclic amino containing 1 to 4 hetero atoms of one or two species selected from nitrogen, sulfur and oxygen atoms, in addition to one nitrogen atom and carbon atoms. Specific examples are pyrrolidin-1-yl, piperidino, piperazin-1-yl, morpholino, thiomorpholino, tetrahydroazepin-1-yl, etc.

In the "optionally substituted 5- to 7-membered saturated cyclic amino" described above, the "substituents" may be 1 to 3 and include, for example, C₁₋₆ alkyl (e.g., methyl, ethyl, propyl, isopropyl, butyl, isobutyl, sec-butyl, tert-butyl, pentyl, hexyl, etc.), C₆₋₁₄ aryl (e.g., phenyl, 1-naphthyl, 2-naphthyl, 2-biphenylyl, 3-biphenylyl, 4-biphenylyl, 2-anthryl, etc.), C₁₋₆ alkyl-carbonyl (e.g., acetyl, propionyl, etc.), a 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.), oxo, and the like.

In the "optionally substituted heterocyclic group" represented by R¹⁰, R¹¹, R¹², R¹⁴, R¹⁵, R¹⁶ or R¹⁷," the "heterocyclic group" includes, for example, a monovalent group formed by removing any one hydrogen atom from a 5- to 14-membered (mono-, bi- or tricyclic) hetero ring containing 1 to 4 hetero atoms of one or two species selected from nitrogen, sulfur and oxygen atoms, in addition to the carbon atoms, preferably (i) a 5- to 14-membered (preferably 5- to 10-membered) aromatic hetero ring, (ii) a 5- to 10-membered non-aromatic hetero ring, or (iii) a 7-to 10-membered bridged-hetero ring.

Specific examples of the "5- to 14-membered (preferably 5- to 10-membered) aromatic hetero ring include aromatic hetero rings such as thiophene, benzo[b]thiophene, benzo[b]furan, benzimidazole, benzoxazole, benzothiazole, benzisoxazole, naphtho[2, 3-b]thiophene, furan, pyrrole, imidazole, pyrazole, pyridine, pyrazine, pyrimidine, pyridazine, indole, isoindole, 1H-indazole, purine, 4H-quinolidine, isoquinoline, quinoline, phthalazine, naphthylidine, quinoxaline, quinazoline, cinnoline, carbazole, β-carboline, phenanthridine, acridine, phenazine, thiazole, isothiazole, phenothiazine, isoxazole, furazane, phenoxazine, etc., or rings formed by condensing one or more of these rings (preferably a monocyclic ring) with one or more (preferably 1 or 2) aromatic rings (e.g., benzene ring, etc.), and the like.

The "5- to 10-membered non-aromatic hetero ring" described above includes, for example, pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole, and the like.

The "7- to 10-membered bridged-hetero ring includes, for example, quinuclidine, 7-azabicyclo[2.2.1]heptane, and the like.

The "heterocyclic group" is a 5- to 14-membered (preferably 5- to 10-membered) (monocyclic or bicyclic) heterocyclic group containing preferably 1 to 4 hetero atoms of one or two species selected from nitrogen, sulfur and oxygen atoms, in addition to the carbon atoms. Specific examples are an aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc., a non-aromatic heterocyclic group such as 1-pyrolidinyl,2-pyrolidinyl,3-pyrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, etc.

Among them, more preferred are 5- or 6-membered heterocyclic groups containing 1 to 3 hetero atoms selected from nitrogen, sulfur and oxygen atom in addition to the carbon atoms, and the like. Specific examples include 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, etc.

The "substituent" in the "optionally substituted heterocyclic group" includes the same substituents exemplified as "substituents" given for the "optionally substituted hydrocarbon group" represented by R¹⁰, R¹¹, R¹², R¹⁴, R¹⁵, R¹⁶ or R¹⁷ described above.

The "heterocyclic group" may have, for example, 1 to 5, preferably 1 to 3, of the above substituents at substitutable positions. When the number of substituents is 2 or more, the respective substituents may be the same or different.

The "C₁₋₆ alkyl" represented by R¹³ includes, for example, methyl, ethyl, propyl, isopropyl, butyl, isobutyl, sec-butyl, tert-butyl, pentyl, hexyl, and the like.

The "substituent" in the "optionally substituted amino" represented by R¹ includes 1 or 2 of the "substituents" in the "optionally substituted hydrocarbon group" represented by R¹⁰, R¹¹, R¹², R¹⁴, R¹⁵, R¹⁶ or R¹⁷ described above.

In the formula (I), R² represents hydrogen atom or an optionally substituted hydrocarbon group.

The "optionally substituted hydrocarbon group" is the same as those given for the "optionally substituted hydrocarbon group" represented by R¹⁰, R¹¹, R¹², R¹⁴, R¹⁵, R¹⁶ or R¹⁷ described above.

In the formula (I), R³ represents an electron-withdrawing group and includes, for example, a halogen atom (e.g., fluorine, chlorine, bromine, iodine), cyano group, nitro group, a halogenomethyl group (e.g., fluoromethyl, chloromethyl, bromomethyl, iodomethyl), a group represented by formula -S(O)ₚ-R⁴ (wherein p represents 1 or 2, and R⁴ represents hydrogen atom or an optionally substituted hydrocarbon group), a group represented by formula -COOR⁵ (R⁵ represents hydrogen atom or an optionally substituted hydrocarbon group), a group represented by formula -CONR⁶R⁷ (wherein R⁶ and R⁷, which may be the same or different, each represents hydrogen atom or an optionally substituted hydrocarbon group), a group represented by formula -SO₂NH-R⁸ (R⁸ represents hydrogen atom or an optionally substituted hydrocarbon group) or a group represented by formula -CO-R⁹ (R⁹ represents hydrogen atom or an optionally substituted hydrocarbon group), and the like.

The "optionally substituted hydrocarbon group" represented by R⁴, R⁵, R⁶, R⁷, R⁸ or R⁹ includes the same as those given for the "optionally substituted hydrocarbon group" represented by R¹⁰, R¹¹, R¹², R¹⁴, R¹⁵, R¹⁶ or R¹⁷ described above.

In the formula (I), n represents an integer of 0 to 2 and m represents an integer of 1 to 6.

Among those described above, a halogen atom or the "optionally halogenated alkyl" is preferred as R¹, especially a halogen atom.

The symbol n showing the number of substituents for R¹ is preferably 0 or 1.

Among those described above, a hydrogen atom or an optionally substituted alkyl group (e.g., C₁₋₆ alkyl such as methyl, ethyl, propyl, isopropyl, butyl, isobutyl, sec-butyl, tert-butyl, pentyl, hexyl, etc.) are preferred as R².

The substituent in the "optionally substituted alkyl group" includes the substituents" given for the "optionally substituted hydrocarbon group" represented by R¹⁰, R¹¹, R¹², R¹⁴, R¹⁵, R¹⁶ or R¹⁷ described above. The number of substituents is preferably 1 to 3.

In the "optionally substituted alkyl group," preferred substituents are (1) hydroxy, (2) amino, (3) mono-C₁₋₆ alkylamino (e.g., methylamino, ethylamino, etc.), (4) di-C₁₋₆ alkylamino (e.g., dimethylamino, diethylamino, ethylmethylamino, etc.), (5) carbamoyl, (6) mono-C₁₋₆ alkyl-carbamoyl (e.g., methylcarbamoyl, ethylcarbamoyl, etc.), (7) di-C₁₋₆ alkyl-carbamoyl (e.g., dimethylcarbamoyl, diethylcarbamoyl, ethylmethylcarbamoyl, etc.), (8) 2-oxo-1,3-dioxolen-4-yl which may be optionally substituted with C₁₋₆ alkyl (e.g., methyl, ethyl, propyl, isopropyl, butyl, isobutyl, sec-butyl, tert-butyl, pentyl, hexyl, etc.), and the like.

For R², hydrogen atom or C₁₋₆ alkyl is particularly preferred.

The number of the methylene chain represented by m is preferably 2.

Among those described above, the group represented by the formula -S(O)ₚ-R⁴ (wherein p is 1 or 2 and R⁴ represents hydrogen atom or an optionally substituted hydrocarbon group) is preferred as R³.

Preferably, p is 2.

For R⁴, an optionally substituted alkyl group (e.g., C₁₋₆ alkyl such as methyl, ethyl, propyl, isopropyl, butyl, isobutyl, sec-butyl, tert-butyl, pentyl, hexyl, etc.) is preferred.

For R⁴, an unsubstituted C₁₋₆ alkyl group is particularly preferred.

In a preferred embodiment, Compound (I) includes, for example, the compounds represented by formula (II) below: 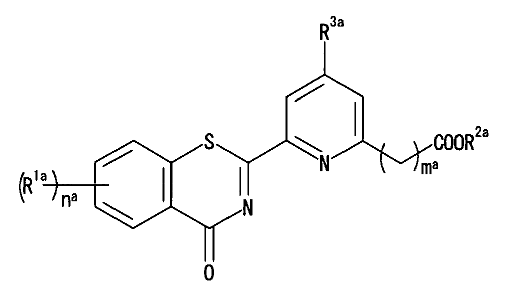 [wherein R^{1a} represents a halogen atom or optionally halogenated alkyl, R^{2a} represents hydrogen atom or C₁₋₆ alkyl group, R^{3a} represents an electron-withdrawing group, n^{a} represents an integer of 0 to 2, and m^{a} represents an integer of 1 to 3 (preferably 2)], or salts thereof, etc.

In the present invention, more preferred compounds are, for example:
tert-butyl
   3-[6-(4-oxo-4H-1,3-benzothiazin-2-yl)-4-(methylsulfonyl)pyridin-2-yl]propanoate;
   3-[6-(4-oxo-4H-1,3-benzothiazin-2-yl)-4-(methylsulfonyl)pyridin-2-yl]propanoic acid;
tert-butyl
   3-[4-(methylthio)-6-(4-oxo-4H-1,3-benzothiazin-2-yl)-2-pyridyl]propanoate;
tert-butyl
   3-[4-(methylsulfinyl)-6-(4-oxo-4H-1,3-benzothiazin-2-yl)-2-pyridyl]propanoate;
   3-[4-(methylsulfinyl)-6-(4-oxo-4H-1,3-benzothiazin-2-yl)-2-pyridyl]propanoic acid;
tert-butyl
   3-[6-(7-chloro-4-oxo-4H-1,3-benzothiazin-2-yl)-4-(methylsulfonyl)pyridin-2-yl]prop anoate;
   3-[6-(7-chloro-4-oxo-4H-1,3-benzothiazin-2-yl)-4-(methylsulfonyl)pyridin-2-yl]prop anoic acid;
tert-butyl
   3-[6-(7-fluoro-4-oxo-4H-1,3-benzothiazin-2-yl)-4-(methylsulfonyl)pyridin-2-yl]prop anoate;
   3-[6-(7-fluoro-4-oxo-4H-1,3-benzothiazin-2-yl)-4-(methylsulfonyl)pyridin-2-yl]prop anoic acid;
tert-butyl
   3-[6-(7-methyl-4-oxo-4H-1,3-benzothiazin-2-yl)-4-(methylsulfonyl)pyridin-2-yl]pro panoate;
   3-[6-(7-methyl-4-oxo-4H-1,3-benzothiazin-2-yl)-4-(methylsulfonyl)pyridin-2-yl]pro panoic acid;
ethyl
   3-[6-(7-chloro-4-oxo-4H-1,3-benzothiazin-2-yl)-4-(methylsulfonyl)pyridin-2-yl]prop anoate;
methyl
   3-[6-(7-chloro-4-oxo-4H-1,3-benzothiazin-2-yl)-4-(methylsulfonyl)pyridin-2-yl]prop anoate;
propyl
   3-[6-(7-chloro-4-oxo-4H-1,3-benzothiazin-2-yl)-4-(methylsulfonyl)pyridin-2-yl]prop anoate;
   (5-methyl-2-oxo-1,3-dioxolen-4-yl)methyl
   3-[6-(7-chloro-4-oxo-4H-1,3-benzothiazin-2-yl)-4-(methylsulfonyl)pyridin-2-yl]prop anoate;
   2,3-dihydroxypropyl
   3-[6-(7-chloro-4-oxo-4H-1,3-benzothiazin-2-yl)-4-(methylsulfonyl)pyridin-2-yl]prop anoate;
   2-(diethylamino)ethyl
   3-[6-(7-chloro-4-oxo-4H-1,3-benzothiazin-2-yl)-4-(methylsulfonyl)pyridin-2-yl]prop anoate; and,
   2-(diethylamino)-2-oxoethyl
   3-[6-(7-chloro-4-oxo-4H-1,3-benzothiazin-2-yl)-4-(methylsulfonyl)pyridin-2-yl]prop anoate;
   or their salts.

In the present invention, particularly preferred compounds are, for example:
3-[6-(4-oxo-4H-1,3-benzothiazin-2-yl)-4-(methylsulfonyl)pyridin-2-yl]propanoic acid;
3-[6-(7-chloro-4-oxo-4H-1,3-benzothiazin-2-yl)-4-(methylsulfonyl)pyridin-2-yl]prop anoic acid;
3-[6-(7-fluoro-4-oxo-4H-1,3-benzothiazin-2-yl)-4-(methylsulfonyl)pyridin-2-yl]prop anoic acid;
3-[6-(7-methyl-4-oxo-4H-1,3-benzothiazin-2-yl)-4-(methylsulfonyl)pyridin-2-yl]pro panoic acid;
2-(diethylamino)ethyl 3-[6-(7-chloro-4-oxo-4H-1,3-benzothiazin-2-yl)-4-(methylsulfonyl)pyridin-2-yl]prop anoate; and
2-(diethylamino)-2-oxoethyl 3-[6-(7-chloro-4-oxo-4H-1,3-benzothiazin-2-yl)-4-(methylsulfonyl)pyridin-2-yl]prop anoate.

Salts of Compound (I) and its intermediates include, for example, metal salts, ammonium salts, salts with organic bases, salts with inorganic acids, salts with organic acids, salts with basic or acidic amino acids, etc. Where these salts are used for medical purpose, these salts are preferably pharmacologically acceptable salts. Preferred examples of the metal salts include alkali metal salts such as sodium salts, potassium salts; alkaline earth metal salts such as calcium salts, magnesium salts, barium salts, etc.; aluminum salts, and the like. Preferred examples of the salts with organic bases include salts with trimethylamine, triethylamine, tromethamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc.

Preferred examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of the salts with basic amino acids include salts with arginine, lysine, ornithine, etc. Preferred examples of the salts with acidic amino acids include salts with aspartic acid, glutamic acid, etc.

Among them, preferred are pharmaceutically acceptable salts. For example, where the compounds have acidic functional groups therein, inorganic salts such as alkali metal salts (e.g., sodium salts, potassium salts, etc.), alkaline earth metal salts (e.g., calcium salts, magnesium salts, barium salts, etc.), ammonium salts, etc. are preferred; where the compounds have basic functional groups therein, preferred are salts with inorganic acids such as hydrobromic acid, nitric acid, sulfuric acid, phosphoric acid, etc., or salts with organic acids such as acetic acid, phthalic acid, fumaric acid, oxalic acid, tartaric acid, maleic acid, citric acid, succinic acid, methanesulfonic acid, p-toluenesulfonic acid, etc.

Compound (I) may be any of hydrates and non-hydrates. Examples of the hydrates are 0.5 hydrate, 1 hydrate, 1.5 hydrate and 2 hydrate, etc.

Where Compound (I) is obtained as a mixture of optically active substances (racemates), the mixture can be resolved into the desired (R)- and (S)-forms by means of publicly known optical resolution.

The prodrug of Compound (I) is used to mean a compound which is converted into Compound (I) upon interaction with enzymes or gastric juice, etc. under physiological conditions in vivo, namely, a compound which is converted into Compound (I) upon enzymatic oxidation, reduction, hydrolysis, etc., or a compound which is converted into Compound (I) upon hydrolysis, etc. by gastric juice, etc. The prodrugs of Compound (I) include compounds wherein amino of Compound (I) is acylated, alkylated or phosphorylated [e.g., the amino in Compound (I) is substituted with eicosanoyl, alanyl, pentylaminocarbonyl, (5-methyl-2-oxo-1,3-dioxolen-4-yl)-methoxycarbonyl, tetrahydrofuranyl, pyrrolidylmethyl, pivaloyloxymethyl, tert-butyl, etc.], compounds wherein hydroxy of Compound (I) is acylated, alkylated, phosphorylated, borated, etc. [e.g., compounds wherein hydroxy of Compound (I) is substituted with acetyl, palmitoyl, propanoyl, pivaloyl, succinyl, fumaryl, alanyl, dimethylaminomethylcarbonyl, etc.], or compounds wherein carboxy in Compound (I) is esterified or amidated [e.g., compounds wherein carboxy in Compound (I) is substituted with ethyl, phenyl, carboxymethyl, dimethylaminomethyl, pivaloyloxymethyl, ethoxycarbonyloxyethyl, phthalidyl, (5-methyl-2-oxo-1,3-dioxolen-4-yl)methyl, cyclohexyloxycarbonylethyl, methylamide, etc.], and so on. These compounds can be manufactured from Compound (I) by per se known methods.

The prodrugs of Compound (I) may be compounds which are converted into Compound (I) under the physiological conditions as described in "Pharmaceutical Research and Development", vol. 7, Drug Design, pages 163-198, published 1990 by Hirokawa Publishing Co.

Processes for producing Compound (I) are described below.

Compound (I) can be prepared by the process shown by reaction equation 1 below or with modifications.

Each symbol for the compounds in the following reaction equation 1 has the same significance as defined above. The compounds in the reaction equation further include their salts; examples of the salts are the same as those given for the salts of Compound (I).

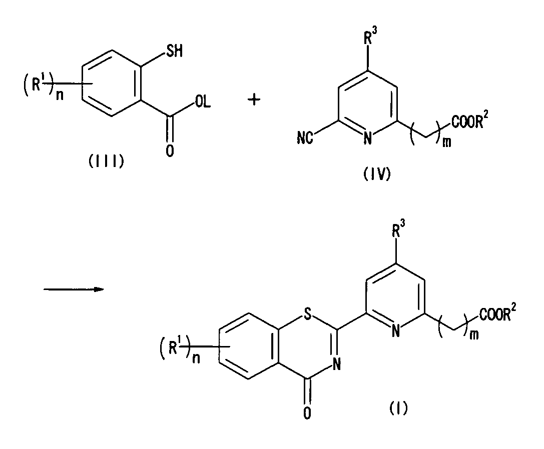

Compound (I) can be obtained by reacting Compound (III) with Compound (IV).

In Compound (III), L represents hydrogen atom or a splitting-off group. Examples of the splitting-off group include alkyl (e.g., C₁₋₆ alkyl such as methyl, ethyl, etc.), etc.

The reaction can be carried out in the presence of a base.

Compound (IV) is used in an amount of approximately 0.4 to 2 mol, preferably approximately 0.8 to 1.5 mol, based on 1 mol of Compound (III).

The base is used in an amount of approximately 1 to 3.0 mol, preferably approximately 1 to 2.0 mol, based on 1 mol of Compound (III).

Examples of the "base" include inorganic bases such as sodium carbonate, potassium carbonate, cesium carbonate, etc.; aromatic amines such as pyridine, lutidine, etc.; tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine, etc.; alkali metal hydrides such as sodium hydride, potassium hydride, etc.; metal amides such as sodium amide, lithium diisopropylamide, lithium hexamethyldisilazide, etc.; metal alkoxides such as sodium methoxide, sodium ethoxide, potassium tert-butoxide, etc.

It is advantageous to perform the reaction in the absence of any solvent or in the presence of a solvent inert to the reaction. The solvent is not particularly limited so far as the reaction proceeds, but examples of the solvent used are aromatic amines, halogenated hydrocarbons, aliphatic hydrocarbons, aromatic hydrocarbons, ethers, amides or a mixture of two or more of these solvents, etc. Among them, pyridine, toluene, etc. are preferred. When the reaction is carried out in, e.g., pyridine, the base is not necessarily required.

The reaction temperature may be normally about 100 to 150°C, preferably 120 to 130°C. When the reaction is carried out at the boiling point of a solvent used, the reaction time may be usually about 3 to 72 hours, preferably about 8 to 24 hours.

The reaction is carried out preferably under reflux at the boiling point of a solvent used.

Where Compound (III) is commercially available, the commercial product can be used as it is; alternatively, Compound (III) can also be produced by per se known processes or their modifications. For example, the objective Compound (III) can be produced from the anthranylic acids corresponding to Compound (III) by publicly known processes (e.g., Journal of Organic Chemistry, 18, 1380, 1953, etc.) or from the salicylic acids corresponding to Compound (III) by publicly known processes (e.g., Journal of Organic Chemistry, 31, 3980, 1966, etc.).

Where Compound (IV) is commercially available, the commercial product can be used as it is; alternatively, Compound (IV) can also be produced by per se known processes or their modifications. For example, Compound (III) can be produced from the pyridine derivatives corresponding to Compound (IV) in Compound (IV) by publicly known processes (e.g., Journal of Organic Chemistry, 18, 1380, 1953, etc.) or from the salicylic acids corresponding to Compound (IV) by publicly known processes (e.g., Journal of Organic Chemistry, 48, 1375, 1983; Synthesis, 316, 316, 1983, etc.).

In the reaction described above, where the starting compound contains amino, carboxy and hydroxy as the substituents, these groups may be protected by protecting groups generally used in peptide chemistry, etc., and the objective compound can be obtained, if necessary, by removing these protecting groups after the reaction.

As protecting groups for the amino, there are used, for example, formyl or optionally substituted C₁₋₆ alkyl-carbonyl (e.g., acetyl, propionyl, etc.), phenylcarbonyl, C₁₋₆ alkoxy-carbonyl (e.g., methoxycarbonyl, ethoxycarbonyl, etc.), phenyloxycarbonyl, C₇₋₁₀ aralkyloxy-carbonyl (e.g., benzyloxycarbonyl, etc.), trityl or phthaloyl, etc. As the substituents for these groups, there are used a halogen atom (e.g., fluorine, chlorine, bromine, iodine, etc.), C₁₋₆ alkyl-carbonyl (e.g., acetyl, propionyl, valeryl, etc.), nitro, etc. The number of substituents is 1 to 3.

As protecting groups for the carboxy, there are used, for example, optionally substituted C₁₋₆ alkyl (e.g., methyl, ethyl, propyl, isopropyl, butyl, tert-butyl, 2-trimethyl, etc.), phenyl, trityl or silyl, etc. As the substituents for these groups, there are used a halogen atom (e.g., fluorine, chlorine, bromine, iodine, etc.), formyl, C₁₋₆ alkyl-carbonyl (e.g., acetyl, propionyl, butylcarbonyl, etc.), nitro, C₁₋₆ alkyl (e.g., methyl, ethyl, tert-butyl, etc.), C₆₋₁₀ aryl (e.g., phenyl, naphthyl, etc.), etc. The number of substituents is 1 to 3.

As protecting groups for the hydroxy, there are used, for example, optionally substituted C₁₋₆ alkyl (e.g., methyl, ethyl, propyl, isopropyl, butyl, tert-butyl, etc.), phenyl, C₇₋₁₁ aralkyl (e.g., benzyl, etc.), formyl, C₁₋₆ alkyl-carbonyl (e.g., acetyl, propionyl, etc.), phenyloxycarbonyl, C₇₋₁₁ aralkyloxy-carbonyl (e.g., benzyloxycarbonyl, etc.), tetrahydropyranyl, tetrahydrofuranyl or silyl, etc. As the substituents for these groups, there are used a halogen atom (e.g., fluorine, chlorine, bromine, iodine, etc.), C₁₋₆ alkyl (e.g., methyl, ethyl, tert-butyl, etc.), C₇₋₁₁ aralkyl (e.g., benzyl, etc.), C₆₋₁₀ aryl (e.g., phenyl, naphthyl, etc.), nitro, etc. The number of substituents is 1 to 4.

To remove the protecting groups, per se known methods or their modifications are used. For example, a method for treating with an acid, a base, ultraviolet, hydrazine, phenylhydrazine, sodium N-methyldithiocarbamate, tetrabutylammonium fluoride, palladium acetate, etc. and the like as well as reducing reactions are used.

In any case, publicly known deprotecting reactions, acylation, alkylation, hydrogenation, oxidization, reduction, carbon chain elongation and substituent-exchanging reaction may be employed alone or in combination of two or more to synthesize Compound (I). The methods described in, e.g., SHIN-JIKKENKAGAKU-KOZA, Vols. 14 and 15, 1977 (MARUZEN Publishing Co.) can be adopted for these reactions.

Examples of the "aromatic amines" described above are pyridine, lutidine, quinoline, etc.

Examples of the "halogenated hydrocarbons" described above are dichloromethane, chloroform, 1,2-dichloroethane, carbon tetrachloride, etc.

Examples of the "aliphatic hydrocarbons" described above are hexane, pentane, cyclohexane, etc.

Examples of the "aromatic hydrocarbons" described above are benzene, toluene, xylene, chlorobenzene, etc.

Examples of the "ethers" described above are diethyl ether, diisopropyl ether, diphenyl ether, tetrahydrofuran, dioxane, 1,2-dimethoxyethane, etc.

Examples of the "amides" described above are N,N-dimethylformamide, N,N-dimethylacetamide, hexamethylphosphoric triamide, etc.

The desired product yielded by the reactions described above in a free form may be converted into a salt in a conventional manner. Alternatively, when the product yielded in a salt form may be converted into a free form or other salts in a conventional manner. Compound (I) thus obtained is isolated and purified from the reaction solution by known techniques such as partition, concentration, solvent extraction, fractional distillation, crystallization, recrystallization, chromatography, and the like.

When Compound (I) exists as configurational isomers, diastereomers, conformers, etc., each can be isolated, if necessary, by any of the isolation or separation techniques described above. When Compound (I) is a racemate, it can be separated into (S) and (R) forms, by a conventional means of optical resolution.

Where stereoisomers of Compound (I) exist, these isomers, either alone or in a mixture thereof, are also within the scope of the present invention.

Compound (I) of the present invention is capable of binding to MIF and is thus not only useful as the MIF binding agent available as various diagnostic agents or reagents, but also possesses an excellent cytoprotective and cell death inhibitory activities on animals, especially mammal (e.g., human, monkey, dog, cat, rabbit, guinea pig, rat, mouse, etc.). For example, Compound (I) inhibits oxidative stress-induced cell death, serum-deprived cell death, cell death induced by growth factor deficiency, cell death induced by HMG-CoA reductase inhibitors, cell death induced by anticancer agents, NO-mediated cell death, cell death induced by amyloid β protein, etc.

Compound (I) of the present invention capable of binding to MIF has effects of promoting the expression of genes under control of ARE (e.g., genes of factors for protecting cells from various stresses; etc.), effects of upregulating (promoting) the production or activation of gene proteins (gene products) under control of ARE, etc. and is also useful as an ARE activator.

As the genes under control of ARE, there are heme oxygenase-1, liver glutathione S-transferase Ya subunit, liver glutathione S-transferase Yc subunit, glutathione S-transferase Yb subunit, glutathione S-transferase Yc1 subunit, gamma-glutamylcysteine synthetase, NAD(P)H:quinone reductase, UDP-glucuronosyltransferase, exon 1, bilirubin-specific UDP-glucuronosyltransferase, NAD(P)H-menadione oxidoreductase, etc.

As such, Compound (I) of the present invention capable of binding to MIF can increase the factors for protecting cells from stress, thereby to strongly inhibit cell death induced by various causes and protect cells.

In Compound (I) of the present invention, the group represented by -(CH₂)ₘ-COOR² (wherein the symbols have the same significance as defined above) is introduced at the specific position of its main skeleton below, and Compound (I) has markedly excellent pharmacokinetics (oral absorption, etc.). 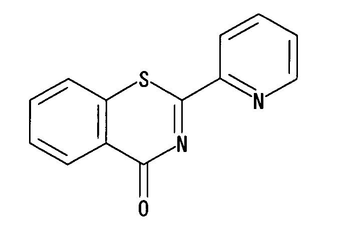 In addition, R³ (electron-withdrawing group) is further introduced at a specific position for substitution so that Compound (I) exhibits the effects of markedly reducing side effects (PPARα activation effects inducing carcinogenicity, hepatomegaly, etc.), which were often observed with known 1,3-benzothiazinone derivatives, while maintaining its pharmacological activities.

Therefore, Compound (I) is low toxic and can be used as a safe medicament, and is useful as an agent for the prevention/treatment of, e.g., circulatory disorders, bone/joint disorders, infectious disorders, inflammatory disorders, renal disorders, central nervous disorders, cancer or diabetes mellitus later described, an anti-aging agent, etc.

The circulatory disorders include, for example, cardiomyopathy (e.g., congestive cardiomyopathy, hypertrophic obstructive cardiomyopathy, hypertrophic nonobstructive cardiomyopathy, idiopathic cardiomyopathy, constrictive cardiomyopathy, diabetic cardiomyopathy, etc.), heart failure (e.g., chronic heart failure, chronic congestive heart failure, acute heart failure, cardiac decompensation, left heart failure, right heart failure, congestive heart failure, acute congestive heart failure, metabolic heart failure, diastolic heart failure, high-output heart failure, low-output heart failure, refractory heart failure, poor prognosis of myocardial infarction, etc.), angina pectoris, myocardial infarction, arterial sclerosis (aneurysm, coronary arteriosclerosis, cerebral arteriosclerosis, peripheral arteriosclerosis, etc.), postischemic reperfusion injury, etc.

The bone/joint disorders include, for example, rheumatoid arthritis, osteoarthritis, knee osteoarthritis, etc.

The infectious disorders include, for example, viral infections such as cytomegalovirus, influenza virus, herpes virus, etc., rickettsial infections, bacterial infections, etc.

The inflammatory disorders include, for example, diabetic complications such as retinopathy, nephropathy, neuropathy, macroangiopathy, etc.; arthritis such as rheumatoid myelitis, periostitis, etc.; postoperative/posttraumatic inflammation; remission of swelling; pharyngitis; urocystitis; pneumonia; atopic dermatitis; inflammatory bowel disorders such as Crohn's disease, ulcerative colitis, etc.; meningitis; inflammatory oculopathy; inflammatory pulmonary diseases such as pneumonia, silicosis, pulmonary sarcoidosis, pulmonary tuberculosis, etc.

The renal disorders include, for example, ischemic acute renal failure, hemolytic uremic syndrome, acute tubular necrosis, hydronephrosis, nephritis, glomerulonephritis, glomerulosclerosis, renal failure, thrombotic microangiopathy, kidney transplant rejection, complication of dialysis, organ failure including radiation-induced nephropathy, etc.

The central nervous disorders include, for example, neurodegenerative disorders (e.g., Alzheimer's disease, Parkinson's disease, Huntington's disease, polyglutamine disease, amyotrophic lateral sclerosis, AIDS encephalopathy, etc.), central nervous system disorders (disorders such as cerebral hemorrhage and cerebral infarction and its sequelae/complications, head injury, spinal injury, cerebral edema, sensory impairment, sensory dysfunction, autonomic impairment, autonomic dysfunction, multiple sclerosis, etc.), dementia, memory impairment, impaired consciousness, amnesia, anxiety symptom, stress symptom, uncomfortable mental status, peripheral neuropathy, etc.

The cancer includes, for example, colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.

The diabetes mellitus includes, for example, type I diabetes mellitus, type II diabetes mellitus, or its complications (diabetic retinopathy, diabetic cataract, diabetic nephropathy, diabetic neuropathy, etc.), etc.

Where Compound (I) is used as the preventive and/or therapeutic agent described above, the compound can be administered either orally or parenterally according to publicly known methods. Compound (I) is mixed with pharmaceutically acceptable carriers and orally administered generally in the form of solid preparations such as tablets, capsules, granules, powders, etc., or parenterally, i.e., intravenously, subcutaneously, intramuscularly, etc. in the form of injections, or suppositories, buccals, etc. The compound may also be administered sublingually, subcutaneously, intramuscularly, etc., in the form of sustained-release preparations such as buccals, microcapsules, etc.

The dose of Compound (I) may vary depending upon subject to be administered, route of administration or conditions but is not particularly limited. For the treatment of, e.g., heart failure, a single dose for an adult patient is usually about 0.1 to 50 mg/kg, preferably about 0.1 to 10 mg/kg, more preferably about 0.1 to 1.0 mg/kg. The dose is usually divided and administered about 1 to 3 times per day.

Compound (I) is contained in the pharmaceutical composition of the present invention in an amount of about 0.01 to 100% by weight, based on the total weight of the pharmaceutical composition.

The pharmaceutically acceptable carriers described above include a wide variety of organic or inorganic carrier materials conventionally used for pharmaceutical preparations, and are formulated as, for example, excipients, lubricants, binders, disintegrants in solid preparations; solvents, dissolution aids, suspending agents, isotonizing agents, buffers, soothing agents, etc. in liquid preparations. If necessary, additives for pharmaceutical preparations such as preservatives, antioxidants, coloring agents, sweeteners, etc. can also be used.

Preferred examples of the excipients described above include lactose, sucrose, D-mannitol, starch, crystalline cellulose, light silicic anhydride, etc. Preferred examples of the lubricants described above include magnesium stearate, calcium stearate, talc, colloidal silica, etc. Preferred examples of the binders described above include crystalline cellulose, sucrose, D-mannitol, dextrin, hydroxypropyl cellulose, hydroxypropylmethyl cellulose, polyvinyl pyrrolidone, etc. Preferred examples of the disintegrants described above include starch, carboxymethyl cellulose, calcium carboxymethyl cellulose, sodium croscarmellose, sodium carboxymethyl starch, etc. Preferred examples of the solvents described above include water for injection, alcohol, propylene glycol, Macrogol, sesame oil, corn oil, etc. Preferred examples of the dissolution aids described above include polyethylene glycol, propylene glycol, D-mannitol, benzyl benzoate, ethanol, trisaminomethane, cholesterol, triethanolamine, sodium carbonate, sodium citrate, etc. Preferred examples of the suspending agents described above include surfactants such as stearyl triethanolamine, sodium laurylsulfate, lauryl aminopropionate, lecithin, benzalkonium chloride, benzetonium chloride, glycerin monostearate, etc.; hydrophilic polymers such as polyvinyl alcohol, polyvinyl pyrrolidone, sodium carboxymethyl cellulose, methyl cellulose, hydroxymethyl cellulose, hydroxyethyl cellulose, hydroxypropyl cellulose, etc. Preferred examples of the isotonizing agents described above include sodium chloride, glycerin, D-mannitol, etc. Preferred examples of the buffers described above include buffers such as phosphates, acetates, carbonates, citrates, etc. Preferred examples of the soothing agents described above include benzyl alcohol, etc. Preferred examples of the preservatives described above include para-oxybenzoic acid esters, chlorobutanol, benzyl alcohol, phenethyl alcohol, dehydroacetic acid, sorbic acid, etc. Preferred examples of the antioxidants described above include sulfites, ascorbic acid, etc.

By adding to Compound (I) a suspending agent, a dissolution aid, a stabilizer, an isotonizing agent, a preservative etc., the compound can be prepared as an intravenous, subcutaneous or intramuscular injection in a conventional manner. In such cases, the compound can be freeze-dried, if necessary, in a conventional manner. In administration to, e.g., human, the compound of the present invention can be safely administered orally or parenterally, directly as such or in the form of a pharmaceutical composition prepared by mixing the compound with a pharmacologically acceptable carrier, excipient and diluent chosen appropriately.

The pharmaceutical compositions described above include oral preparations (e.g., powders, granules, capsules, tablets), injections, drip infusions, topical preparations (e.g., nasal preparations, transdermal preparations, etc.), suppositories (e.g., rectal suppositories, vaginal suppositories) and the like.

These preparations can be manufactured in a conventional manner generally used in pharmaceutical making processes.

An injection can be produced by dissolving, suspending or emulsifying Compound (I) together with a dispersing agent (e.g., Tween 80 (manufactured by Atlas Powder Company, USA), HCO 60 (manufactured by Nikko Chemicals), polyethylene glycol, carboxymethyl cellulose, sodium alginate, etc.), a preservative (e.g., methyl paraben, propyl paraben, benzyl alcohol, etc.), an isotonizing agent (e.g., sodium chloride, mannitol, sorbitol, glucose, etc.) and other additives to prepare an aqueous injection, or by dissolving, suspending or emulsifying in a vegetable oil such as olive oil, sesame oil, cottonseed oil, corn oil, etc., propylene glycol or the like to prepare an oily injection.

An oral preparation can be produced in a conventional manner by adding to Compound (I) an excipient (e.g., lactose, sucrose, starch, etc.), a disintegrant (e.g., starch, calcium carbonate, etc.), a binder (e.g., starch, gum arabic, carboxymethyl cellulose, polyvinylpyrrolidone, hydroxypropyl cellulose, etc.), a lubricant (e.g., talc, magnesium stearate, polyethylene glycol 6000, etc.) and other additives, compressing the resulting mixture and, if necessary, coating the compressed product for the purpose of taste masking, enteric degradation or sustained release by techniques per se publicly known. Coating agents for this purpose include, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by Rohm Company, Germany, methacrylic acid/acrylic acid copolymer) and dyes (e.g., iron oxide, titanium dioxide, etc.). In an enteric preparation, for the purpose of separation of the two phases an intermediate phase may be provided between the enteric phase and the drug-containing phase in a conventional manner.

A topical preparation can be prepared in a conventional manner by compounding Compound (I) or its salt as a solid, semi-solid or liquid topical composition. The solid composition described above is prepared by, for example, powdering Compound (I) or its salt as it is or in a mixture with an excipient (e.g., glycol, mannitol, starch, microcrystalline cellulose, etc.), a thickening agent (e.g., natural rubber, cellulose derivatives, acrylic polymer, etc.) and other additives to make the powdery composition. The liquid composition described above is prepared into an oily or aqueous suspension in almost the same manner as with the injectable preparation. The semi-solid composition is preferably an aqueous or oily gel, or an ointment. All of these compositions may contain a pH adjuster (e.g., carbonic acid, phosphoric acid, citric acid, hydrochloric acid, sodium hydroxide, etc.), a preservative (e.g., paraoxybenzoic acid esters, chlorobutanol, benzalkonium chloride, etc.) and other additives.

In the form of, e.g., a suppository, Compound (I) is prepared into an oily or aqueous solid, semi-solid or liquid composition by techniques per se publicly known. Oily bases used for the composition described above include glycerides of higher fatty acids [e.g., cacao butter, uitepsols (manufactured by Dynamite Nobel Company, Germany), etc.], moderate fatty acids [e.g., MIGLYOL (manufactured by Dynamite Nobel Company, Germany), etc.], vegetable oils (e.g., sesame oil, soybean oil, cottonseed oil, etc.), and the like. Aqueous bases include, for example, polyethylene glycols and propylene glycol. Bases for aqueous gels include, for example, natural rubbers, cellulose derivatives, vinyl polymers, acrylic polymers, etc.

The other drugs to be used in combination with Compound (I) includes, for example, those given below. These drugs can be administered orally or parenterally (e.g., as nasal preparations, injections, suppositories, etc.). Alternatively, these drugs may be formulated in a single preparation or may be mixed with pharmaceutically acceptable carriers, excipients, binders, diluents, etc., which can be administered individually or simultaneously. When these drugs are individually formulated in the respective preparations, the individual preparations may be administered by mixing them using, e.g., a diluent when administered; or, individual preparations may also be administered to the one and same subject, separately or simultaneously or with time intervals.

Examples of drugs which provide a synergistic effect by concomitant use with Compound (I) include cardiotonic agents (e.g., cardiotonic glycosides such as digoxin, etc., β agonists such as dopamine, dobutamine, etc., phosphodiesterase inhibitors such as amrinone, milrinone, etc.); antiarrhythmic drugs (e.g., class I antiarrhythmic drugs such as disopyramide, lidocaine, procainamide, etc., class III antiarrhythmic drugs such as amiodarone, sotalol, etc., β blockers such as propranolol, etc.); vasodilators (e.g., angiotensin convertase inhibitors such as captopril, enalapril, etc., nitrate drugs such as nitroprusside, isosorbide dinitrate, etc., calcium receptor antagonists such as verapamil, diltiazem, nicardipine, nifedipine, etc., angiotensin II receptor antagonists such as losartan, candesartan, etc.; diuretic agents (e.g., loop diuretics such as furosemide, bumetamide, etc., thiazide diuretics such as chlorothiazide, bendrofluazide, etc., potassium-sparing diuretic agents such as amiloride, spironolactone, etc.) and the like.

Furthermore, when Compound (I) is used in combination with HMG-CoA reductase inhibitors (e.g., simvastatin, atorvastatin, etc.), fibrate-type antihyperlipidemic drugs (e.g., gemfibrozil, etc.), anticancer agents (e.g., ifosfamide, UFT, adriamycin, doxorubicin, peplomycin, cisplatin, cyclophosphamide, 5-FU, methotrexate, mitomycin C, mitoxantrone, etc.) or the like, side effects by the HMG-CoA reductase inhibitors, fibrate-type antihyperlipidemic drugs, anticancer agents, etc., which give damages to normal cells, are alleviated.

### EXAMPLES

Hereinafter, the present invention will be described more specifically with reference to REFERENCE EXAMPLES, EXAMPLES, PREPARATION EXAMPLES and EXPERIMENTAL EXAMPLES but is not deemed to be limited thereto.

In REFERENCE EXAMPLES and EXAMPLES below, % refers to percent by weight unless otherwise indicated.

¹H-NMR spectra were measured by Mercury-300 (300 MHz) or Bruker AVANCE DPX-300 (300 MHz) type spectrometer using tetramethylsilane as an internal standard. All values are shown in ppm.

The melting point was determined using a Buchi B-545 δ melting point apparatus.

The other symbols used herein have the following definitions.
- s:: singlet
- d:: doublet
- dd:: double doublet
- t:: triplet
- dt:: double triplet
- q:: quartet
- m:: multiplet
- br:: broad
- brs:: broad singlet
- J:: coupling constant
- Hz:: Hertz
- DMF:: N,N-dimethylformamide
- THF:: tetrahydrofuran
- CDCl₃:: deuterated chloroform
- DMSO-d₆:: dimethylsulfoxide-d₆
- NaH:: sodium hydride
- WSC:: 1-ethyl-3-(dimethylaminopropyl)carbodiimide hydrochloride
- HOBt·H₂O:: 1-hydroxy-1H-benzotriazole monohydrate
- DIBAL:: diisobutylaluminum hydride

The term room temperature is used to mean the range from about 10 to 35°C in general, but is not to be construed as strictly limitative.

### REFERENCE EXAMPLE 1

### Methyl 4-chloropyridine-2-carboxylate

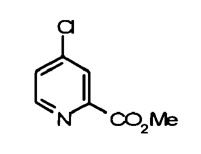

DMF (3.5 ml, 45 mmol) was dropwise added slowly to thionyl chloride (105 ml, 1.47 mol) previously warmed at 40-42°C and then picolinic acid (35.0 g, 284 mmol) was added thereto by small portions over 10 minutes or longer. The reaction temperature was maintained at 40-45°C for 15 minutes and then gradually elevated to 72°C by heating, and the mixture was agitated at the same temperature for 22 hours. An excess of thionyl chloride was concentrated under reduced pressure and the residue was diluted with toluene (100 ml). Under ice cooling, a mixture of methanol (25 ml) and toluene (20 ml) was dropwise added to the solution. The reaction mixture was allowed to stand overnight, neutralized with an aqueous sodium bicarbonate solution and then extracted with ethyl acetate. The extract was washed with saturated sodium chloride aqueous solution and dried over anhydrous magnesium sulfate. The solvent was then concentrated under reduced pressure. The residue was purified (eluted with hexane -ethyl acetate 2:1-3:2, v/v) by column chromatography using silica gel (300 g) to give the title compound (38.7 g, 79%) as crystals.
¹H-NMR (CDCl₃) δ: 4.03 (3H, s), 7.50 (1H, dd, J=5.4 Hz, 2.1 Hz), 8.15 (1H, d, J=2.1 Hz), 8.66 (1H, d, J=5.4 Hz).

### REFERENCE EXAMPLE 2

### 4-Chloropyridine-2-carbaldehyde

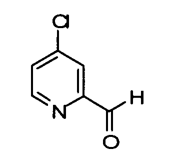

Methyl 4-chloropyridine-2-carboxylate (3.42 g, 20.0 mmol) was dissolved in THF (100 ml) and DIBAL (1.5M toluene solution, 20.0 ml, 30.0 mmol) was dropwise added to the solution in a nitrogen flow at -70°C (dry ice - acetone). After stirring t -70°C for an hour, water (100 ml) was portionwise added to the reaction mixture. After reverting to room temperature, the mixture was extracted with ethyl acetate (100 ml x 2). The extracts were combined and washed with saturated aqueous sodium chloride solution (100 ml). After drying over anhydrous magnesium sulfate, the solvent was concentrated under reduced pressure. The residue was purified (eluted with n-hexane-ethyl acetate = 3:1, v/v) by silica gel column chromatography to give the title compound (2.10 g, 74%) as crystals.
¹H-NMR (CDCl₃) δ: 7.54 (1H, dd, J=1.8 Hz, 5.1 Hz), 7.96 (1H, d, J=1.8 Hz), 8.70 (1H, d, J=5.1 Hz), 10.06 (1H, s).

### REFERENCE EXAMPLE 3

### Tert-butyl 3-(4-chloropyridin-2-yl)acrylate

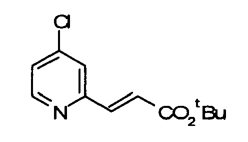

NaH (60% oily, 0.68 g, 17.0 mmol) was suspended in THF (100 ml) and tert-butyl diethylphosphonoacetate (3.73 g, 14.8 mmol) was dropwise added to the suspension at room temperature. After stirring for an hour, a solution of 4-chloropyridine-2-carbaldehyde (2.00 g, 14.1 mmol) in THF (10 ml) was dropwise added to the reaction mixture, followed by stirring for further 1 hour. The solvent was concentrated under reduced pressure and water (100 ml) was slowly added to residue. The mixture was then extracted with ethyl acetate (100 ml x 2). After washing with saturated sodium chloride aqueous solution (100 ml), the extract was dried over anhydrous magnesium sulfate and the solvent was concentrated under reduced pressure. The residue was purified (eluted with n-hexane-ethyl acetate = 3:1, v/v) by column chromatography using silica gel to give the title compound (3.20 g, 95%) as crystals.
¹H-NMR (CDCl₃) δ: 1.53 (9H, s), 6.84 (1H, d, J=23.4 Hz), 7.26 (1H, m), 7.42 (1H, m), 7.51 (1H, d, J=23.4 Hz), 8.52 (1H, d, J=7.2 Hz).

### REFERENCE EXAMPLE 4

### Tert-butyl 3-(4-chloropyridin-2-yl)propanoate

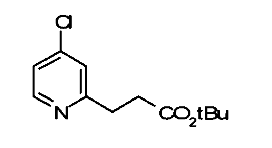

Bismuth chloride (32.7 g, 104 mmol) was added to a solution of tert-butyl 3-(4-chloropyridin-2-yl)acrylate (9.95 g, 41.5 mmol) in ethanol (200ml), and sodium borohydride (7.85 g, 208 mmol) was portionwise added to the solution under ice cooling. After stirring at the same temperature for 18 hours, bismuth chloride was removed by filtration through celite and the filtrate was concentrated under reduced pressure. Water (300 ml) was gradually added to the residue and the mixture was extracted with ethyl acetate (300 ml x 2). After washing with saturated sodium chloride aqueous solution (300 ml), the extract was dried over anhydrous sodium sulfate and the solvent was concentrated under reduced pressure. The residue was purified (eluted with n-hexane-ethyl acetate = 15:1-3:1, v/v) by column chromatography using silica gel to give the title compound (6.11 g, 61%) as a colorless oil.
¹H-NMR (CDCl₃) δ: 1.41 (9H, s), 2.70 (2H, t, J=7.5 Hz), 3.05 (2H, t, J=7.5 Hz), 7.12 (1H, dd, J=1.8 Hz, 5.4 Hz), 7.20 (1H, d, J=1.8 Hz), 8.40(1H, d, J=5.1 Hz).

### REFERENCE EXAMPLE 5

### Tert-butyl 3-(4-chloropyridin-2-yl)propanoate

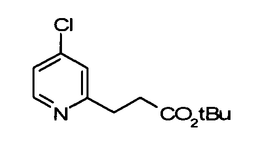

Tert-butyl 3-(4-chloropyridin-2-yl)acrylate (16.7 g, 69.7 mmol) was dissolved in ethanol (600 ml), followed by hydrogenation at room temperature under normal pressure in the presence of Raney nickel (25 g). The catalyst was removed by filtration through celite and the filtrate was concentrated under reduced pressure. The residue was purified (eluted with n-hexane-ethyl acetate = 10:1-8:1, v/v) by column chromatography using silica gel to give the title compound (12.6 g, 75%) as a colorless oil.

### REFERENCE EXAMPLE 6

### Tert-butyl 3-(4-chloro-1-oxidopyridin-2-yl)propanoate

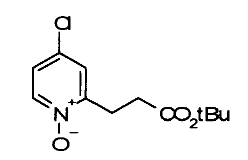

After tert-butyl 3-(4-chloropyridin-2-yl)propanoate (12.6 g, 52.2 mmol) was dissolved in ethyl acetate (500 ml), 3-chloroperbenzoic acid (containing 65%, 13.9 g, 52.2 mmol) was added to the solution under ice cooling. After stirring at room temperature for 24 hours, the reaction mixture was partitioned between ethyl acetate (300 ml) and 0.33N sodium hydroxide aqueous solution (300 ml). The organic layer was fractionated and washed again in 0.33N sodium hydroxide aqueous solution (150 ml). The wash solution was extracted with ethyl acetate (500 ml). The extracts were combined, washed with saturated sodium chloride aqueous solution (500 ml) and dried over anhydrous sodium sulfate. The residue was purified (eluted with hexane-ethyl acetate = 5:1-0:1, v/v) by column chromatography using silica gel to give the title compound (13.3 g, 99%) as crystals.
¹H-NMR (CDCl₃) δ: 1.42 (9H, s), 2.74 (2H, t, J=6.9 Hz), 3.13 (2H, t, J=6.9 Hz), 7.14 (1H, dd, J=3.0 Hz, 6.9 Hz), 7.30 (1H, d, J=3.0 Hz), 8.16 (1H, d, J=6.9 Hz).

### REFERENCE EXAMPLE 7

### Tert-butyl 3-(4-chloro-6-cyanopyridin-2-yl)propanoate

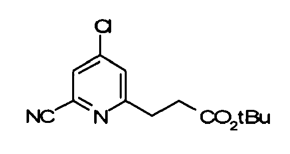

After tert-butyl 3-(4-chloro-1-oxidopyridin-2-yl)propanoate (13.5 g, 52.2 mmol) was dissolved in acetonitrile (300 ml), trimethylsilyl cyanide (25.9 g, 260 mmol) and N,N-dimethylcarbamoyl chloride (6.73 g, 62.6 mmol) in this order were dropwise added to the solution. After stirring at 90°C for 5 hours with heating, the reaction mixture was concentrated under reduced pressure. The residue was purified (eluted with hexane-ethyl acetate = 10:1, v/v) by column chromatography using silica gel to give the title compound (12.6 g, 91%) as a light brown oil.
¹H-NMR (CDCl₃) δ: 1.43 (9H, s), 2.73 (2H, t, J=6.9 Hz), 3.09 (2H, t, J=6.9 Hz), 7.43 (1H, d, J=1.8 Hz), 7.52 (1H, d, J=1.8 Hz).

### REFERENCE EXAMPLE 8

### Tert-butyl 3-[6-cyano-4-(methylthio)pyridin-2-yl]propanoate

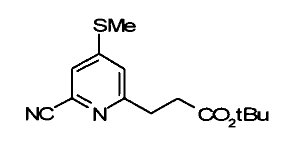

After tert-butyl 3-(4-chloro-6-cyanopyridin-2-yl)propanoate (12.6 g, 47.4 mmol) was dissolved in THF (500 ml), sodium methanethiolate (8.3 g, 119.0 mmol) was added to the solution and the mixture was stirred under
a nitrogen atmosphere at 80°C for 3 hours. The reaction mixture was concentrated under reduced pressure and water (300 ml) was added to the residue, followed by extraction with ethyl acetate (300 ml x 2). The extract was washed with saturated sodium chloride aqueous solution (300 ml). After drying over anhydrous sodium sulfate, the solvent was concentrated under reduced pressure. The residue was purified (eluted with hexane-ethyl acetate = 5:1, v/v) by column chromatography using silica gel to give the title compound (12.8 g, 97%) as an oil.
¹H-NMR (CDCl₃) δ: 1.43 (9H, s), 2.50 (3H, s), 2.70 (2H, t, J=6.9 Hz), 3.04 (2H, t, J=6.9 Hz), 7.14 (1H, d, J=1.5 Hz), 7.28 (1H, d, J=1.5 Hz).

### REFERENCE EXAMPLE 9

### Tert-butyl 3-[6-cyano-4-(methylsulfonyl)pyridin-2-yl] propanoate

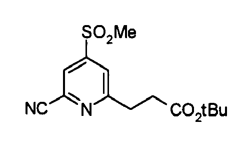

After tert-butyl 3-[6-cyanl-4-(methylthio)pyridin-2-yl]propanoate (12.8 g, 46.0 mmol) was dissolved in ethyl acetate (500 ml), 3-chloroperbenzoic acid (containing 65%, 24.4 g, 92.0 mmol) was added to the solution under ice cooling. After stirring at room temperature for 3 hours, ethyl acetate (300 ml) was added to the reaction mixture, followed by washing with 0.5N sodium hydroxide aqueous solution (300 ml x 2). The wash solution was again extracted with ethyl acetate (500 ml). The extracts were combined, washed with saturated sodium chloride aqueous solution (500 ml) and dried over anhydrous sodium sulfate. The solvent was concentrated under reduced pressure. The residue was purified (eluted with hexane-ethyl acetate = 3:1-1:1, v/v) by column chromatography using silica gel to give the title compound (2.9 g, 90%) as white crystals.
¹H-NMR (CDCl₃) δ: 1.43 (9H, s), 2.80 (2H, t, J=6.9 Hz), 3.11 (3H, s), 3.25 (2H, t, J=6.9 Hz), 7.90 (1H, d, J=1.5 Hz), 7.97 (1H, d, J=1.5 Hz).

### REFERENCE EXAMPLE 10

### Tert-butyl 3-(1-oxido-4-methylthiopyridin-2-yl)propanoate

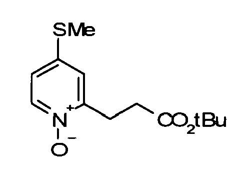

After tert-butyl 3-(4-chloro-1-oxidopyridin-2-yl)propanoate (1.0 g, 4.0 mmol) was dissolved in THF (30 ml), sodium methanethiolate (1.3 g, 18.0 mmol) was added to the solution and the mixture was stirred under a nitrogen atmosphere at 80°C for 3 hours. The reaction mixture was concentrated under reduced pressure and water (100 ml) was added thereto, followed by extraction with ethyl acetate (100 ml x 2). The extract was washed with saturated sodium chloride aqueous solution (100 ml). After drying over anhydrous sodium sulfate, the solvent was concentrated under reduced pressure to give the title compound (0.79 g, 74%) as amorphous.
¹H-NMR (CDCl₃) δ: 1.41 (9H, s), 2.50 (3H, s), 2.75 (2H, t, J=7.2 Hz), 3.15 (2H, t, J=7.2 Hz), 6.94-6.98 (1H, m), 7.09 (1H, d, J=2.7Hz), 8.11 (1H, d, J=6.9Hz).

### REFERENCE EXAMPLE 11

### Tert-butyl 3-[6-cyano-4-(methylthio)pyridin-2-yl]propanoate

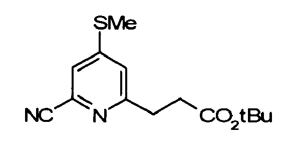

After tert-butyl 3-(1-oxido-4-methothiopyridin-2-yl)propanoate (1.59 g, 5.9 mmol) was dissolved in acetonitrile (200 ml), trimethylsilyl cyanide (1.8 g, 17.7 mmol) and N,N-dimethylcarbamoyl chloride (0.76 g, 7.1 mmol) were dropwise added thereto in this order. The reaction mixture was stirred at 80°C for 2.5 hours and then concentrated under reduced pressure. The residue was purified (eluted with hexane-ethyl acetate = 3:1, v/v) by column chromatography using silica gel to give the title compound (1.2 g, 71%) as a light brooil.

### REFERENCE EXAMPLE 12

### 4-Chloro-2-mercaptobenzoic acid

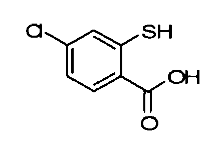

While maintaining the reaction temperature at 0-5°C, 1N sodium hydroxide aqueous solution (310 ml) of 4-chloroanthranilic acid (52.0 g, 302 mmol) and sodium nitrate (21.2 g, 306 mmol) was dropwise added slowly to a mixture of conc. hydrochloric acid (90 ml) and ice (100 g). The reaction mixture was stirred at 0°C for 30 minutes and then neutralized by adding potassium acetate thereto. This aqueous solution was slowly added to a mixture of O-ethyl potassium dithiocarbonate (97.0 g, 600 mmol) and water (100 ml), which had been previously heated at 80°C, while keeping the reaction temperature between 75 and 80°C. After stirring at 80°C for 30 minutes, the reaction mixture was cooled back to room temperature and acidified (pH of about 3) with conc. hydrochloric acid, whereby an oily substance was precipitated. The oily substance was isolated by decantation of the aqueous layer. 10% Sodium hydroxide aqueous solution (300 ml) was added thereto. After stirring at 80°C for 2 hours, sodium hydrosulfite (30 g) was added to the mixture and stirring was continued at 80°C for further 10 minutes. The reaction mixture was filtered, and the filtrate was cooled and acidified (pH of about 4) with conc. hydrochloric acid to give a light yellow solid. The solid was filtered off and dissolved in ethyl acetate (1000 ml). After washing with water, the solution was dried over anhydrous sodium sulfate and concentrated under reduced pressure to give the title compound (55.6 g, 98%) as crystals.
¹H-NMR (CDCl₃) δ :4.76 (1H, s), 7.17 (1H, dd, J=1.8 Hz, 8.7 Hz), 7.36 (1H, d, J=1.8 Hz), 8.06 (1H, d, J=8.7 Hz).

### REFERENCE EXAMPLE 13

### Methyl 4-chloro-2-mercaptobenzoate

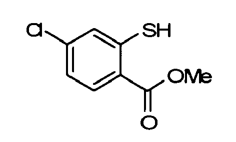

4-Chloro-2-mercaptobenzoic acid (17.5 g, 92.8 mmol) and a solution of 10% hydrogen chloride in methanol (360 ml) were stirred at 60°C for 22 hours. After the reaction mixture was concentrated under reduced pressure, the residue was purified (eluted with n-hexane) by column chromatography using silica gel (100 g) to give the title compound (15.3 g, 81%) as yellow crystals.
¹H-NMR (CDCl₃) δ: 3.93 (3H, s), 4.88 (1H, s), 7.11-7.15 (1H, m), 7.32-7.33 (1H, m), 7.95 (1H, dd, J=1.2 Hz, 8.4 Hz).

### REFERENCE EXAMPLE 14

### Methyl 4-fluoro-2-mercaptobenzoate

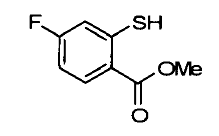

4-Fluoro-2-mercaptobenzoic acid (6.11 g, 35.5 mmol) and a methanol solution (70 ml) of 10% hydrogen chloride were stirred at 60°C for 18 hours. After the reaction mixture was concentrated under reduced pressure, the residue was purified (eluted with n-hexane-ethyl acetate = 4:1, v/v) by column chromatography using silica gel (150 g) to give the title compound (5.26 g, 80%) as crystals.
¹H-NMR (CDCl₃) δ: 3.92 (3H, s), 5.00 (1H, s), 6.81-6.91 (1H, m), 7.03 (1H, dd, J=2.5, 9.2 Hz), 8.04 (1H, dd, J=6.0, 8.9 Hz).

### REFERENCE EXAMPLE 15

### Methyl 4-methyl-2-mercaptobenzoate

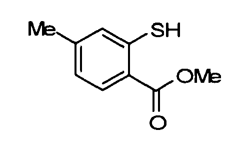

4-Methyl-2-mercaptobenzoic acid (8.41 g, 50.0 mmol) and a methanol solution (100 ml) of 10% hydrogen chloride were stirred at 60°C for 18 hours. After the reaction mixture was concentrated under reduced pressure, the residue was purified (eluted with n-hexane-ethyl acetate = 4:1, v/v) by column chromatography using silica gel (150 g) to give the title compound (6.52 g, 72%) as crystals.
¹H-NMR (CDCl₃) δ: 2.32 (3H, s), 3.90 (3H, s), 4.75 (1H, s), 6.96 (1H, d, J=8.1 Hz), 7.12 (1H, s), 7.91 (1H, d, J=8.1 Hz).

### REFERENCE EXAMPLE 16

### Ethyl 3-(4-chloropyridin-2-yl)acrylate

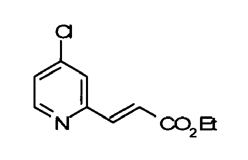

While stirring, ethyl phosphonoacetate (44.6 g, 199 mmol) was dropwise added to a suspension ofNaH (60% oily, 9.04 g, 226 mmol) in THF (500 ml) at room temperature. After stirring for an hour, a solution of 4-chloropyridine-2-carbaldehyde (24.6 g, 174 mmol) in THF (100 ml) was dropwise added to the reaction mixture followed by stirring for an hour. After water (500 ml) was slowly added to the reaction mixture, the mixture was extracted with ethyl acetate (1000 ml x 2). The extract was washed with saturated sodium chloride aqueous solution (500ml) and dried over anhydrous sodium sulfate. The solvent was then concentrated under reduced pressure. The residue was purified (eluted with n-hexane-ethyl acetate = 10:1, v/v) by column chromatography using silica gel to give the title compound (34 g, 92%) as crystals.
¹H-NMR (CDCl₃) δ: 1.34 (3H, t, J=7.2 Hz), 4.27 (2H, q, J=7.2 Hz), 6.93 (1H, d, J=15.6 Hz), 7.25-7.28 (1H, m), 7.41 (1H, d, J=1.8 Hz), 7.60 (1H, d, J=15.6 Hz), 8.52 (1H, d, J=5.4 Hz).

### REFERENCE EXAMPLE 17

### Ethyl 3-(4-chloropyridin-2-yl)propanoate

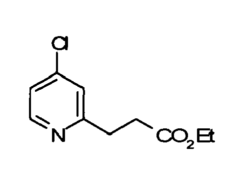

Ethyl 3-(4-chloropyridin-2-yl)acrylate (34.0 g, 161 mmol) was dissolved in ethanol (700ml), followed by hydrogenation at room temperature under normal pressure in the presence of Raney nickel (20 g). The catalyst was removed by filtration and the filtrate was concentrated under reduced pressure. The residue was purified (eluted with n-hexane-ethyl acetate = 10:1-8:1, v/v) by column chromatography using silica gel to give the title compound (32.6 g, 95%) as a colorless oil.
¹H-NMR (CDCl₃) δ: 1.23 (3H, t, J=7.2 Hz), 2.79 (2H, t, J=7.5 Hz), 3.09 (2H, t, J=7.5 Hz), 4.12 (2H, q, J=7.2 Hz), 7.12-7.14 (1H, m), 7.21 (1H, d, J=1.8 Hz), 8.40 (1H, d, J=5.1 Hz).

### REFERENCE EXAMPLE 18

### Ethyl 3-(4-chloro 1-oxidopyridin-2-yl)propanoate

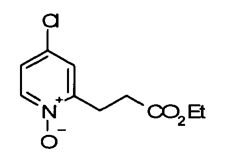

Ethyl 3-(4-chloropyridin-2-yl)propanoate (11.1 g, 52.0 mmol) was dissolved in ethyl acetate (500ml) and 3-chloroperbenzoic acid (containing 65%, 13.8 g, 52.0 mmol) was added to the solution in an ice-bath. After stirring at room temperature for 24 hours, ethyl acetate (300ml) was added to the reaction mixture and the mixture was washed twice with 0.33N sodium hydroxide aqueous solution. After the aqueous layer was extracted with ethyl acetate (500ml), it was combined with the organic layer. After washing with saturated sodium chloride aqueous solution (500ml) and drying over anhydrous sodium sulfate, the solvent was concentrated under reduced pressure to give the title compound (12.0 g, 99%) as an oily substance. ¹H-NMR (CDCl₃) δ: 1.24 (3H, t, J=7.4 Hz), 2.84 (2H, t, J=6.8 Hz), 3.17 (2H, t, J=6.8 Hz), 4.13 (2H, q, J=7.2 Hz), 7.15 (1H, dd, J=2.8 Hz, 7.0 Hz), 7.33 (1H, d, J=2.8 Hz), 8.16 (1H, d, J=7.0 Hz).

### REFERENCE EXAMPLE 19

### Ethyl 3-(4-chloro-6-cyanopyridin-2-yl)propanoate

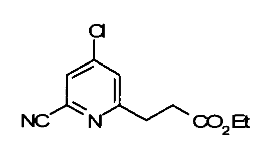

After ethyl 3-(4-chloro-1-oxidopyridin-2-yl)propanoate (12.2 g, 52.0 mmol) was dissolved in acetonitrile (300 ml), trimethylsilyl cyanide (25.9 g, 260 mmol) and N,N-dimethylcarbamoyl chloride (6.73 g, 62.6 mmol) were dropwise added in this order. After stirring at 90°C for 4 hours, the reaction mixture was concentrated under reduced pressure. The residue was purified (eluted with hexane-ethyl acetate = 10:1, v/v) by column chromatography using silica gel to give the title compound (10.6 g, 85%) as an oil.
¹H-NMR (CDCl₃) δ: 1.25 (3H, t, J=7.2 Hz), 2.83 (2H, t, J=6.9 Hz), 3.13 (2H, t, J=6.9 Hz), 4.13 (2H, q, J=7.2 Hz), 7.44 (1H, d, J=1.5 Hz), 7.52 (1H, d, J=1.5 Hz).

### REFERENCE EXAMPLE 20

### Ethyl 3-[6-cyano-4-(methylthio)pyridin-2-yl]propanoate

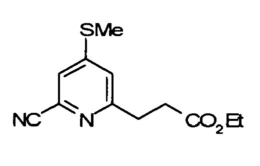

Ethyl 3-(4-chloro-6-cyanopyridin-2-yl)propanoate (9.5 g, 40.04 mmol) was dissolved in THF (500 ml) and sodium methanethiolate (7.0 g, 100 mmol) was added to the solution, followed by stirring under a nitrogen atmosphere at 80°C for 1.5 hours. The reaction mixture was concentrated under reduced pressure and water (300 ml) was added to the residue, followed by extraction with ethyl acetate (300 ml x 2). The extract was washed with saturated sodium chloride aqueous solution (300 ml) and dried over anhydrous sodium sulfate. The solvent was then concentrated under reduced pressure. The residue was purified (eluted with hexane-ethyl acetate = 3:1, v/v) by column chromatography using silica gel to give the title compound (8.8 g, 89%) as white crystals.
¹H-NMR (CDCl₃) δ: 1.25 (3H, t, J=7.2 Hz), 2.51 (3H, s), 2.80 (2H, t, J=7.2 Hz), 3.07 (2H, t, J=7.2 Hz), 4.12 (2H, q, J=7.2 Hz), 7.15 (1H, d, J=1.5 Hz), 7.29 (1H, d, J=1.5 Hz).

### REFERENCE EXAMPLE 21

### Ethyl 3-[6-cyano-4-(methylsulfonyl)pyridin-2-yl] propanoate

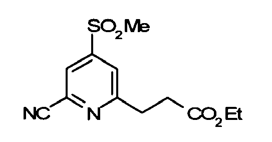

Ethyl 3-[6-cyano-4-(methylthio)pyridin-2-yl] propanoate (7.45 g, 30.0 mmol) was dissolved in ethyl acetate (400 ml), and 3-chloro-perbenzoic acid (containing 65%, 15.8 g, 60.0 mmol) was added to the solution under ice cooling. After stirring at room temperature for 3 hours, ethyl acetate (300 ml) was added to the reaction mixture and the mixture was washed with 0.5N sodium hydroxide aqueous solution (180 ml x 2) and then saturated sodium chloride aqueous solution (300 ml), and dried over anhydrous sodium sulfate, followed by concentration under reduced pressure. The residue was purified (eluted with hexane-ethyl acetate = 1:1-1:2, v/v) by column chromatography using silica gel and recrystallized from ethyl acetate-n-hexane to give the title compound (7.9 g, 93%) as white crystals.
¹H-NMR (CDCl₃) δ: 1.26 (3H, t, J=7.2 Hz), 2.90 (2H, t, J=6.9 Hz), 3.12 (3H, s), 3.28 (2H, t, J=6.9 Hz), 4.13 (2H, q, J=7.2 Hz), 7.92 (1H, d, J=1.5 Hz), 7.98 (1H, d, J=1.5 Hz).

### EXAMPLE 1

### Tert-butyl 3-[6-(4-oxo-4H-1,3-benzothiazin-2-yl)-4-(methylsulfonyl)pyridin-2-yl] propanoate

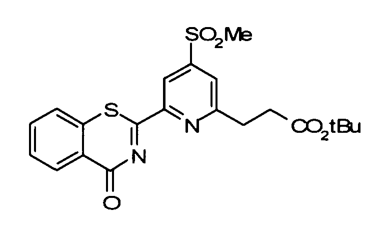

After tert-butyl 3-[6-cyano-4-(methylsulfonyl)pyridin-2-yl] propanoate (1.33 g, 4.3 mmol) and methyl thiosalicylate (1.44 g, 8.6 mmol) were dissolved in toluene (100 ml), triethylamine (1.30 g, 12.9 mmol) was added to the solution, followed by heating to reflux for 24 hours. The reaction mixture was concentrated to approximately half the volume under reduced pressure and the crystals precipitated were taken by filtration. The filtrate was concentrated under reduced pressure and the residue was purified (eluted with hexane-ethyl acetate = 3:1-0:1, v/v) by column chromatography using silica gel. The crystals were combined with those previously obtained and recrystallized from ethyl acetate-n-hexane to give the title compound (10.8 g, 70%) as white crystals.
Melting point: 185.0-186.0°C.
¹H-NMR (CDCl₃) δ: 1.44 (9H, s), 2.70 (2H, t, J=7.4 Hz), 3.16 (2H, t, J=7.3 Hz), 3.37 (3H, s), 7.62-7.75 (3H, m), 8.16 (1H, d, J=0.8 Hz), 8.55-8.59 (1H, m), 8.63 (1H, d, J=0.8 Hz).

### EXAMPLE 2

### 3-[6-(4-Oxo-4H-1,3-benzothiazin-2-yl)-4-(methylsulfonyl)pyridin-2-yl]propanoic acid

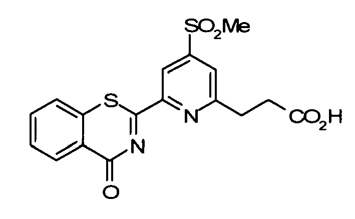

Under ice cooling, tert-butyl 3-[6-(4-oxo-4H-1,3-benzothiazin-2-yl)-4-(methylsulfonyl)pyridin-2-yl] propanoate (1.5 g, 3.4 mmol) was added to trifluoroacetic acid (30 ml). After stirring at room temperature for 30 minutes, the reaction mixture was concentrated under reduced pressure. Diisopropyl ether (150 ml) was added to the residue for crystallization. Recrystallization from ethanol gave the title compound (1.1 g, 85%).
Melting point: 244.5.1-245.5°C
¹H-NMR (DMSO-d₆) δ: 2.75 (2H, t, J=7.2 Hz), 3.15 (2H, t, J=7.1 Hz), 3.45 (3H, s), 7.76-7.79 (1H, m), 7.84-7.89 (1H, m), 7.99 (1H, d, J=7.6 Hz), 8.28 (1H, d, J=0.9 Hz), 8.37-8.40 (1H, m), 8.52 (1H, d, J=0.9 Hz), 12.2 (1H, brs).

| Elemental analysis as C₁₇H₁₄N₂O₅S₂ | | | |
|---|---|---|---|
| Calcd.: | C, 52.30 ; | H, 2.61 ; | N, 7.17 |
| Found: | C, 52.23 ; | H, 2.57 ; | N, 7.09 |

### EXAMPLE 3

### Tert-butyl 3-[4-(methylthio)-6-(4-oxo-4H-1,3-benzothiazin-2-yl)-2-pyridyl] propanoate

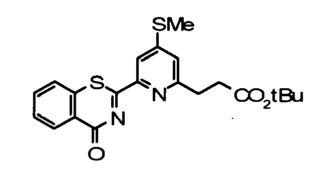

After tert-butyl 3-[6-cyano-4-(methylthio)pyridin-2-yl] propanoate (0.60 g, 2.16 mmol) and methyl thiosalicylate (0.73 g, 4.32 mmol) were dissolved in toluene (50 ml), triethylamine (0.91 ml, 6.48 mmol) was added to the solution followed by heating to reflux for 18 hours. The solvent was concentrated under reduced pressure and the crystals obtained were recrystallized from ethyl acetate-n-hexane to give the title compound (0.40 g, 45%) as light yellow crystals.
Melting point: 155.0-156.0°C.
¹H-NMR (CDCl₃) δ: 1.43 (9H, s), 2.60 (2H, t, J=7.4 Hz), 2.71 (3H, s), 2.94 (2H, t, J=7.5 Hz), 7.28 (1H, s), 7.60-7.70 (3H, m), 8.09 (1H, s), 8.54-8.56 (1H, m).

### EXAMPLE 4

### Tert-butyl 3-[4-(methylsulfinyl)-6-(4-oxo-4H-1,3-benzothiazin-2-yl)-2-pyridyl]propanoate

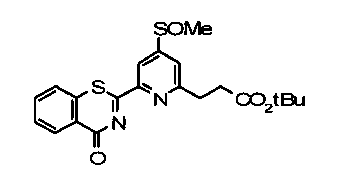

After tert-butyl 3-[4-(methylthio)-6-(4-oxo-4H-1,3-benzothiazin-2-yl)-2-pyridyl]propanoate (0.17 g, 0.41 mmol) was dissolved in chloroform (50 ml), a solution of 3-chloroperbenzoic acid (containing 77%, 0.09 g, 0.41 mmol) in chloroform (10 ml) was dropwise added to the solution. The reaction mixture was stirred at room temperature for an hour, and the solvent was then concentrated under reduced pressure. The crystals obtained were recrystallized from ethyl acetate-n-hexane to give the title compound (0.16 g, 91%) as white crystals.
Melting point: 160.0-160.5°C
¹H-NMR (CDCl₃) δ: 1.44 (9H, s), 2.70 (2H, t, J=7.5 Hz), 2.97 (3H, s), 3.13 (2H, t, J=7.5 Hz), 7.59-7.72 (3H, m), 8.12 (1H, d, J=1.2 Hz), 8.47 (1H, d, J=1.1 Hz), 8.55-8.58 (1H, m).

### EXAMPLE 5

### 3-[4-(Methylsulfinyl)-6-(4-oxo-4H-1,3-benzothiazin-2-yl)-2-pyridyl]propanoic acid

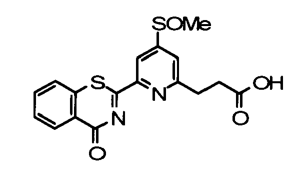

Trifluoroacetic acid (5 ml) was added to tert-butyl 3-[4-(methylsulfinyl)-6-(4-oxo-4H-1,3-benzothiazin-2-yl)-2-pyridyl]propanoate (0.12 g, 0.28 mmol) and the mixture was stirred at room temperature for 30 minutes. Diisopropyl ether was added to the reaction mixture. The precipitates formed were taken by filtration and recrystallized from ethanol to give the title compound (0.095 g, 91%) as white crystals.
Melting point: 265.5-266.0°C
¹H-NMR (DMSO-d₆) δ: 2.73 (2H, t, J=7.3 Hz), 2.93 (3H, s), 3.13 (2H, t, J=7.2 Hz), 7.75-7.78 (1H, m), 7.83-7.88 (1H, m), 7.96 (1H, d, J=7.4 Hz), 8.12 (1H, d, J=1.1 Hz), 8.34-8.39 (2H, m), 12.2 (1H, brs).

### EXAMPLE 6

### Tert-butyl 3-[6-(4-oxo-4H-1,3-benzothiazin-2-yl)-4-(methylsulfonyl)pyridin-2-yl]propanoate

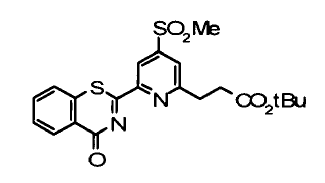

After tert-butyl 3-[4-(methylthio)-6-(4-oxo-4H-1,3-benzothiazin-2-yl)-2-pyridyl]propanoate (0.20 g, 0.48 mmol) was dissolved in chloroform (50 ml), a solution of 3-chloro-perbenzoic acid (containing 77%, 0.22 g, 0.97 mmol) in chloroform (10 ml) was dropwise added thereto. The reaction mixture was stirred at room temperature for 3 hours and the solvent was then concentrated under reduced pressure. The crystals obtained were recrystallized from ethyl acetate-n-hexane to give the title compound (0.061 g, 28%) as white crystals. The ¹H-NMR of this compound coincided with that of the compound obtained in EXAMPLE 1.

### EXAMPLE 7

### Tert-butyl 3-[6-(7-chloro-4-oxo-4H-1,3-benzothiazin-2-yl)-4-(methylsulfonyl)pyridin-2-yl] propanoate

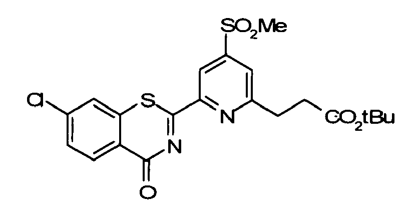

In toluene (400 ml), tert-butyl 3-[6-cyano-4-(methylsulfonyl)pyridin-2-yl] propanoate (10.0 g, 32.2 mmol) and methyl 4-chlorothiosalicylate (9.80 g, 48.3 mmol) were dissolved and triethylamine (6.52 g, 64.4 mmol) was added to the solution, followed by heating to reflux for 24 hours. The reaction mixture was concentrated to approximately 1/4 the volume under reduced pressure and the crystals precipitated were taken by filtration. The filtrate was concentrated under reduced pressure and purified (eluted with hexane-ethyl acetate = 3:1-0:1, v/v) by column chromatography using silica gel. The crystals were combined with those previously obtained and recrystallized from ethyl acetate-n-hexane to give the title compound (10.8 g, 70%).
Melting point: 184.3-184.5°C
¹H-NMR (CDCl₃) δ: 1.43 (9H, s), 2.93 (2H, t, J=7.2 Hz), 3.15 (3H, s), 3.33 (2H, t, J=7.2 Hz), 7.59-7.64 (2H, m), 7.96 (1H, d, J=1.5 Hz), 8.49 (1H, dd, J=0.9 Hz, 8.7 Hz), 8.78 (1H, d, J=1.2 Hz).

| Elemental analysis as C₂₁H₂₁N₂O₅S₂Cl | | | |
|---|---|---|---|
| Calcd.: | C, 52.44 ; | H, 4.40 ; | N, 5.82 |
| Found: | C, 52.41 ; | H, 4.43 ; | N, 5.90 |

### EXAMPLE 8

### 3-[6-(7-Chloro-4-oxo-4H-1,3-benzothiazin-2-yl)-4-(methylsulfonyl)pyridin-2-yl]pro panoic acid

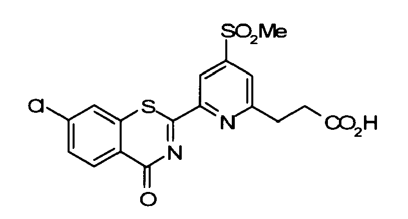

Under ice cooling, trifluoroacetic acid (15 ml) was added to tert-butyl 3-[6-(7-chloro-4-oxo-4H-1,3-benzothiazin-2-yl)-4-(methylsulfonyl)pyridin-2-yl] propanoate (0.97 g, 2.02 mmol). The reaction mixture was stirred at room temperature for 0.5 hours and then concentrated under reduced pressure. The residue was crystallized from diisopropyl ether (25 ml) and then recrystallized from ethanol to give the title compound (0.66 g, 77%).
Melting point: 228.8-228.9°C
¹H-NMR (DMSO-d₆) δ: 2.87 (2H, t, J=7.2 Hz), 3.28 (2H, t, J=7.2 Hz), 3.32 (3H, s), 7.79 (1H, dd, J=1.8 Hz, 8.7 Hz), 8.20-8.21 (2H, m), 8.35 (1H, d, J=8.7 Hz), 8.51 (1H, d, J=1.8 Hz), 12.29 (1H, s).

| Elemental analysis as C₁₇H₁₃N₂O₅S₂Cl | | | |
|---|---|---|---|
| Calcd.: | C, 48.06 ; | H, 3.08 ; | N, 6.59 |
| Found: | C, 48.07 ; | H, 3.06 ; | N, 6.60 |

### EXAMPLE 9

### Tert-butyl 3-[6-(7-fluoro-4-oxo-4H-1,3-benzothiazin-2-yl)-4-(methylsulfonyl)pyridin-2-yl] propanoate

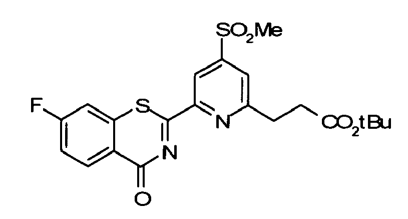

In toluene (50 ml), tert-butyl 3-[6-cyano-4-(methylsulfonyl)pyridin-2-yl] propanoate (1.11 g, 3.6 mmol) and 4-fluoromethyl thiosalicylate (1.0 g, 5.4 mmol) were dissolved and triethylamine (0.72 g, 7.2 mmol) was added thereto, followed by heating to reflux for 24 hours. The reaction mixture was concentrated to approximately 1/5 the volume under reduced pressure and the crystals precipitated were taken by filtration. The filtrate was concentrated under reduced pressure and purified (eluted with hexane-ethyl acetate = 3:1-1:2, v/v) by column chromatography using silica gel. The crystals were combined with those previously obtained and recrystallized from ethyl acetate-n-hexane to give the title compound (1.2 g, 72%). Melting point: 177.6-178.0°C.
¹H-NMR (CDCl₃) δ: 1.44 (9H, s), 2.93 (2H, t, J=7.2 Hz), 3.16 (3H, s), 3.34 (2H, t, J=7.2 Hz), 7.32-7.40 (2H, m), 7.97 (1H, d, J=1.8 Hz), 8.60 (1H, dd, J=5.7 Hz, 8.7 Hz), 8. 80 (1H, d, J=1.5 Hz).

| Elemental analysis as C₂₁H₂₁N₂O₅S₂F | | | |
|---|---|---|---|
| Calcd.: | C, 54.30 ; | H, 4.56 ; | N, 6.03 |
| Found: | C, 54.21 ; | H, 4.57 ; | N, 6.00 |

### EXAMPLE 10

### 3-[6-(7-fluoro-4-oxo-4H-1,3-benzothiazin-2-yl)-4-(methylsulfonyl)pyridin-2-yl]prop anoic acid

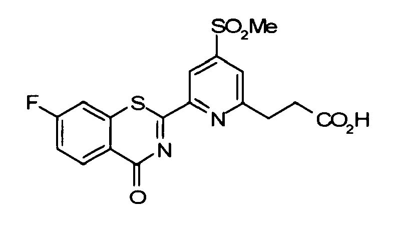

Under ice cooling, trifluoroacetic acid (10 ml) was added to tert-butyl 3-[6-(7-fluoro-4-oxo-4H-1,3-benzothiazin-2-yl)-4-(methylsulfonyl)pyridin-2-yl]prop anoate (1.1 g, 2.4 mmol). The reaction mixture was stirred at room temperature for 0.5 hours and then concentrated under reduced pressure. The residue was crystallized from diisopropyl ether (50 ml) and then recrystallized from ethanol to give the title compound (0.89 g, 90%) as crystals.
Melting point: 235.8-236.2°C
¹H-NMR (DMSO-d₆) δ: 2.87 (2H, t, J=7.2 Hz), 3.28 (2H, t, J=7.2 Hz), 3.44 (3H, s), 7.62 (1H, dt, J=2.4 Hz, 8.7 Hz), 7.99 (1H, dd, J=2.4 Hz, 9. 0 Hz), 8.22 (1H, m), 8.42-8.47 (1H, m), 8.53 (1H, s), 12.35 (1H, s).

| Elemental analysis as C₁₇H₁₃N₂O₅S₂F | | | |
|---|---|---|---|
| Calcd.: | C, 49.99 ; | H, 3.21 ; | N, 6.86 |
| Found: | C, 49.88 ; | H, 3.11 ; | N, 6.82 |

### EXAMPLE 11

### Tert-butyl 3-[6-(7-methyl-4-oxo-4H-1,3-benzothiazin-2-yl)-4-(methylsulfonyl)pyridin-2-yl]pro panoate

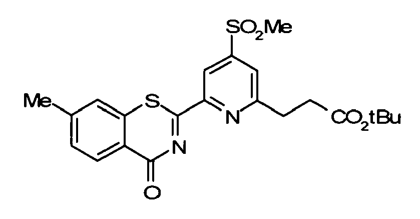

In toluene (50 ml), tert-butyl 3-[6-cyano-4-(methylsulfonyl)pyridin-2-yl]propanoate (1.11 g, 3.6 mmol) and methyl 4-methylthiosalicylate (1.0 g, 5.4 mmol) were dissolved and triethylamine (0.72 g, 7.2 mmol) was added thereto followed by heating to reflux for 24 hours. The reaction mixture was concentrated to approximately 1/5 the volume under reduced pressure and the crystals precipitated were taken by filtration. The filtrate was concentrated under reduced pressure and purified (eluted with hexane-ethyl acetate = 3:1-1:2, v/v) by column chromatography using silica gel. The crystals were combined with those previously obtained and recrystallized from ethyl acetate-n-hexane to give the title compound (1.3 g, 80%).
Melting point: 178.0-178.4°C
¹H-NMR (CDCl₃) δ: 1.43 (9H, s),2.52 (3H, s), 2.93 (2H, t, J=7.2 Hz), 3.16 (3H, s), 3.33 (2H, t, J=7.2 Hz), 7.44-7.49 (2H, m), 7.95 (1H, d, J=1.4 Hz), 8.45 (1H, d, J=8.2 Hz), 8.81 (1H, d, J=1.4 Hz).

| Elemental analysis as C₂₂H₂₄N₂O₅S₂ | | | |
|---|---|---|---|
| Calcd.: | C, 57.37 ; | H, 5.25 ; | N, 6.08 |
| Found: | C, 57.16; | H, 5.08 ; | N, 5.94 |

### EXAMPLE 12

### 3-[6-(7-Methyl-4-oxo-4H-1,3-benzothiazin-2-yl)-4-(methylsulfonyl)pyridin-2-yl]pro panoic acid

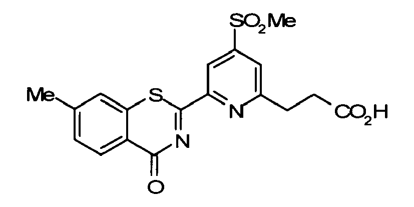

Under ice cooling, trifluoroacetic acid (10 ml) was added to tert-butyl 3-[6-(7-methyl-4-oxo-4H-1,3-benzothiazin-2-yl)-4-(methylsulfonyl)pyridin-2-yl]pro panoate (1.3 g, 2.8 mmol). The reaction mixture was stirred at room temperature for 0.5 hours and then concentrated under reduced pressure. Diisopropyl ether (50 ml) was added to the residue for crystallization. The crystals were recrystallized from ethanol to give the title compound (1.0 g, 93%).
Melting point: 238.9-239.1°C
¹H-NMR (DMSO-d₆) δ: 2.47 (3H, s), 2.88 (2H, t, J=7.2 Hz), 3.28 (2H, t, J=7.2 Hz), 3.43 (3H, s), 7.58 (1H, d, J=8.1 Hz), 7.78 (1H, s), 8.20 (1H, d, J=1.8 Hz), 8.27 (1H, dd, J=1.8 Hz, 8.1 Hz), 8.52 (1H, s), 12.33 (1H, s).

| Elemental analysis as C₁₈H₁₆N₂O₅S₂ | | | |
|---|---|---|---|
| Calcd.: | C, 53.45 ; | H, 3.99 ; | N, 6.93 |
| Found: | C, 53.38 ; | H, 3.97 ; | N, 6.85 |

### EXAMPLE 13

### Ethyl 3-[6-(7-chloro-4-oxo-4H-1,3-benzothiazin-2-yl)-4-(methylsulfonyl)pyridin-2-yl]prop anoate

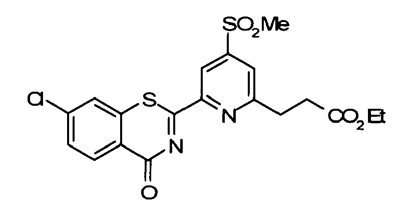

In toluene (200 ml), ethyl 3-[6-cyano-4-(methylsulfonyl)pyridin-2-yl]propanoate (2.79 g, 9.88 mmol) and methyl 4-chlorothiosalicylate (3.0 g, 14.8 mmol) were dissolved and triethylamine (2.01 g, 19.8 mmol) was added to the solution, followed by heating to reflux for 24 hours. The reaction mixture was cooled to room temperature and the crystals precipitated were taken by filtration. The crystals were recrystallized from ethanol to give the title compound (2.72 g, 61%).
Melting point: 240.0-240.2°C
¹H-NMR (CDCl₃) δ: 1.28 (3H, t, J=7.2 Hz), 3.01 (2H, t, J=6.9 Hz), 3.16 (3H, s), 3.38 (2H, t, J=6.9 Hz), 4.17 (2H, q, J=7.2 Hz), 7.59-7.63 (2H, m), 7.97 (1H, s), 8.48 (1H, dd, J=0.9 Hz, 8.1 Hz), 8.78 (1H, d, J=1.8 Hz).

| Elemental analysis as C₁₉H₁₇N₂O₅S₂Cl | | | |
|---|---|---|---|
| Calcd.: | C, 50.38 ; | H, 3.78 ; | N, 6.18 |
| Found: | C, 50.33 ; | H, 3.74 ; | N, 6.16 |

### EXAMPLE 14

### Ethyl 3-[6-(7-chloro-4-oxo-4H-1,3-benzothiazin-2-yl)-4-(methylsulfonyl)pyridin-2-yl]prop anoate

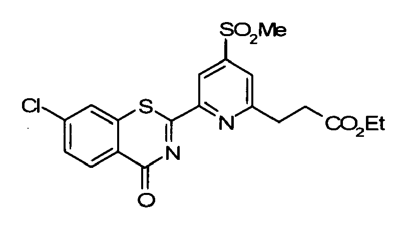

In methylene chloride (5 ml), 3-[6-(7-chloro-4-oxo-4H-1,3-benzothiazin-2-yl)-4-(methylsulfonyl)pyridin-2-yl]prop anoic acid (0.43 g, 1.0 mmol) and triethylamine (0.15 ml, 1.1 mmol) were dissolved, and under ice cooling, ethyl chlorocarbonate (0.10 ml, 1.0 mmol) and 4-dimethylaminopyridine (12 mg, 0.1 mmol) were added to the solution. After stirring at the same temperature for 30 minutes, saturated ammonium chloride aqueous solution (100 ml) was added to the reaction mixture, followed by extraction with methylene chloride (100 ml x 2). The extract was dried over anhydrous sodium sulfate and the solvent was concentrated under reduced pressure. The residue was purified (eluted with hexane-ethyl acetate = 1:1-0:1, v/v) by column chromatography using silica gel and recrystallized from ethanol to give the title compound (0.18 g, 40%). The ¹H-NMR of this compound coincided with that of the compound obtained in EXAMPLE 13.

### EXAMPLE 15

### Ethyl 3-[6-(7-chloro-4-oxo-4H-1,3-benzothiazin-2-yl)-4-(methylsulfonyl)pyridin-2-yl]prop anoate

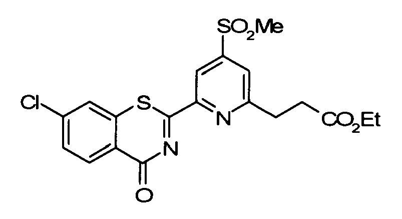

A mixture of 3-[6-(7-chloro-4-oxo-4H-1,3-benzothiazin-2-yl)-4-(methylsulfonyl)pyridin-2-yl]prop anoic acid (0.21 g, 0.5 mmol), ethanol (0.060 ml, 1.0 mmol), HOBt H₂O (92 mg, 0.6 mmol), WSC (116 mg, 0.6 mmol), 4-dimethylaminopyridine (74 mg, 0.6 mmol) and DMF (3 ml) was stirred at room temperature for 96 hours and at 60°C for 24 hours. Water (20 ml) was added to the reaction mixture. The crystals formed were taken by filtration and recrystallized from ethanol to give the title compound (0.12 g, 53%) as crystals. The ¹H-NMR of this compound coincided with that of the compound obtained in EXAMPLE 13.

### EXAMPLE 16

### Ethyl 3-[6-(7-chloro-4-oxo-4H-1,3-benzothiazin-2-yl)-4-(methylsulfonyl)pyridin-2-yl]prop anoate

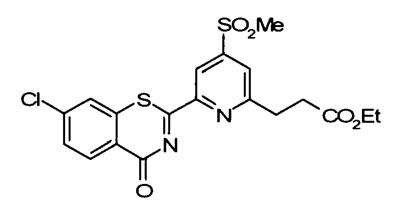

N,N'-Carbonylimidazole (162 mg, 1.0 mmol) was added to a solution of 3-[6-(7-chloro-4-oxo-4H-1,3-benzothiazin-2-yl)-4-(methylsulfonyl)pyridin-2-yl]prop anoic acid (0.21 g, 0.5 mmol) in THF (80 ml), and the mixture was stirred at room temperature for 0.5 hours. After the reaction mixture was concentrated under reduced pressure, ethanol (150 ml) was added to the reaction mixture, followed by heating to reflux for 4.5 hours. The crystals formed were taken by filtration to give the title compound (0.18 g, 80%). The ¹H-NMR of this compound coincided with that of the compound obtained in EXAMPLE 13.

### EXAMPLE 17

### Methyl 3-[6-(7-chloro-4-oxo-4H-1,3-benzothiazin-2-yl)-4-(methylsulfonyl)pyridin-2-yl]prop anoate

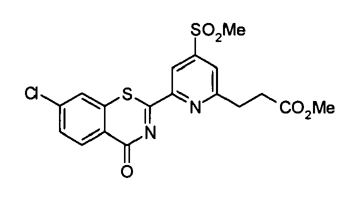

After 3-[6-cyano-4-(methylsulfonyl)pyridin-2-yl]propanoic acid (0.13 g, 0.31 mmol) was dissolved in a solvent mixture of methanol (10 ml) and methylene chloride (15 ml), a diethyl ether solution (1.5 ml, 3.0 mmol) of 2.0 M trimethylsilyl diazomethane was dropwise added to the solution at room temperature over 5 minutes. After stirring for 30 minutes, acetic acid (0.24 g, 4.0 mmol) was added to the reaction mixture and stirring was continued for further 5 minutes. The reaction mixture was concentrated under reduced pressure. The residue was purified (eluted with chloroform) by column chromatography using basic silica gel and further recrystallized from ethanol to give the title compound (0.06 g, 44%) as crystals. Melting point: 203.4-203.6°C
¹H-NMR (CDCl₃) δ: 3.03 (2H, t, J=6.9 Hz), 3.16 (3H, s), 3.40 (2H, t, J=6.9 Hz), 3.74 (3H, s), 7.60-7.64 (2H, m), 7.98 (1H, s), 8.48-8.52 (1H, m), 8.80 (1H, d, J=2.1 Hz).

| Elemental analysis as C₁₈H₁₅N₂O₅S₂Cl | | | |
|---|---|---|---|
| Calcd.: | C, 49.26 ; | H, 3.44 ; | N, 6.38 |
| Found: | C, 49.33 ; | H, 3.52 ; | N, 6.17 |

### EXAMPLE 18

### Methyl 3-[6-(7-chloro-4-oxo-4H-1,3-benzothiazin-2-yl)-4-(methyl sulfonyl)pyridin-2-yl]prop anoate

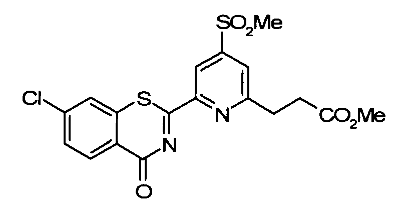

A mixture of 3-[6-(7-chloro-4-oxo-4H-1,3-benzothiazin-2-yl)-4-(methylsulfonyl)pyridin-2-yl]prop anoic acid (0.21 g, 0.5 mmol), methanol (0.041 ml, 1.0 mmol), HOBt H₂O (92 mg, 0.6 mmol), WSC (116 mg, 0.6 mmol), 4-dimethylaminopyridine (74 mg, 0.6 mmol) and DMF (3 ml) was stirred at room temperature for 144 hours and at 60°C for 8 hours. Water (20 ml) was added to the reaction mixture and the crystals formed were taken by filtration. The crystals were recrystallized from ethanol to give the title compound (0.12 g, 55%) as crystals. The ¹H-NMR of this compound coincided with that of the compound obtained in EXAMPLE 17.

### EXAMPLE 19

### Propyl 3-[6-(7-chloro-4-oxo-4H-1,3-benzothiazin-2-yl)-4-(methylsulfonyl)pyridin-2-yl]prop anoate

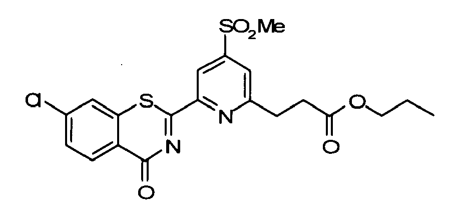

A mixture of 3-[6-(7-chloro-4-oxo-4H-1,3-benzothiazin-2-yl)-4-(methylsulfonyl)pyridin-2-yl]prop anoic acid (0.21 g, 0.5 mmol), 1-propanol (0.30 g, 5.0 mmol), HOBt H₂O (92 mg, 0.6 mmol), WSC (116 mg, 0.6 mmol), 4-dimethylaminopyridine (74 mg, 0.6 mmol) and DMF (3 ml) was stirred at room temperature for 24 hours and at 80°C for 18 hours. Water (20 ml) was added to the reaction mixture and the crystals formed were taken by filtration. The crystals were recrystallized from ethanol to give the title compound (0.06 g, 26%) as crystals.
Melting point: 235.4-235.5°C
¹H-NMR (CDCl₃) δ: 0.93 (3H, t, J=7.5 Hz), 1.60-1.72 (2H, m), 3.03 (2H, t, J=6.9 Hz), 3.17 (3H, s), 3.39 (2H, t, J=6.9 Hz), 4.08 (2H, t, J=6.6 Hz), 7.60-7.64 (2H, m), 7.99 (1H, s), 8.50 (1H, d, J=9.0 Hz), 8.80 (1H, s).

| Elemental analysis as C₂₀H₁₉N₂O₅S₂Cl | | | |
|---|---|---|---|
| Calcd.: | C, 51.44 ; | H, 4. 10 ; | N, 6.00 |
| Found: | C, 51.29 ; | H, 4.05 ; | N, 6.13 |

### EXAMPLE 20

### (5-Methyl-2-oxo-1,3-dioxolen-4-yl)methyl 3-[6-(7-chloro-4-oxo-4H-1,3-benzothiazin-2-yl)-4-(methylsulfonyl)pyridin-2-yl]prop anoate

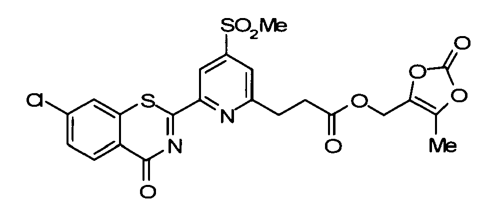

A mixture of 3-[6-(7-chloro-4-oxo-4H-1,3-benzothiazin-2-yl)-4-(methylsulfonyl)pyridin-2-yl]prop anoic acid (0.21 g, 0.5 mmol), (5-methyl-2-oxo-1,3-dioxolen-4-yl)methyl alcohol (0.13 g, 1.0 mmol), HOBt H₂O (92 mg, 0.6 mmol), WSC (116 mg, 0.6 mmol), 4-dimethylaminopyridine (74 mg, 0.6 mmol) and DMF (3 ml) was stirred at room temperature for 24 hours. Water (20 ml) was added to the reaction mixture and the crystals formed were taken by filtration. The crystals were recrystallized from ethanol to give the title compound (0.24 g, 89%) as crystals.
Melting point: 183.1-183.2°C ¹H-NMR (CDCl₃) δ: 2.15 (3H, s), 3.09 (2H, t, J=6.9 Hz), 3.18 (3H, s), 3.40 (2H, t, J=6.9 Hz), 4.86 (2H, s), 7.61 (1H, dd, J=1.2 Hz, 8.7 Hz), 7.66 (1H, d, J=1.8 Hz), 7.97 (1H, d, J=1.2 Hz), 8.49 (1H, d, J=8.7 Hz), 8.80 (1H, d, J=0.9 Hz).

| Elemental analysis as C₂₂H₁₇N₂O₈S₂Cl | | | |
|---|---|---|---|
| Calcd.: | C, 49.21 ; | H, 3.19 ; | N, 5.22 |
| Found: | C, 49.19 ; | H, 3.31 ; | N, 5.18 |

### EXAMPLE 21

### 2,3-Dihydroxypropyl 3-[6-(7-chloro-4-oxo-4H-1,-benzothiazin-2-yl)-4-(methylsulfonyl)pyridin-2-yl]prop anoate

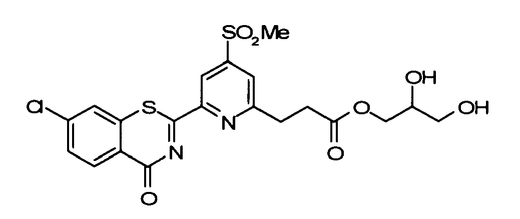

A mixture of 3-[6-(7-chloro-4-oxo-4H-1,3-benzothiazin-2-yl)-4-(methylsulfonyl)pyridin-2-yl]prop anoic acid (0.21 g, 0.5 mmol), glycerol (0.46 g, 5.0 mmol), HOBt H₂O (92 mg, 0.6 mmol), WSC (116 mg, 0.6 mmol), 4-dimethylaminopyridine (74 mg, 0.6 mmol) and DMF (3 ml) was stirred at room temperature for 15 hours. Water (20 ml) was added to the reaction mixture and the crystals formed were taken by filtration. The crystals were recrystallized from ethanol to give the title compound (0.24 g, 89%) as crystals.
Melting point: 240.0-241.0°C.
¹H-NMR (CDCl₃) δ: 2.98 (2H, t, J=7.4 Hz), 3.33-3.43 (4H, m), 3.43 (3H, s), 3.61-3.69 (1H, m), 3.90-4.16 (2H, m), 4.66 (1H, t, J=5.4 Hz), 4.95 (1H, d, J=5.4 Hz), 7.77-7.81 (1H, m), 8.20-8.23 (2H, m), 8.36 (1H, d, J=9.2 Hz), 8.54 (1H, s).

| Elemental analysis as C₂₀H₁₉N₂O₇S₂Cl | | | |
|---|---|---|---|
| Calcd.: | C, 48.14 ; | H, 3.84 ; | N, 5.61 |
| Found: | C, 47.90 ; | H, 3.81 ; | N, 5.55 |

### EXAMPLE 22

### 2-(Diethylamino)ethyl 3-[6-(7-chloro-4-oxo-4H-1,3-benzothiazin-2-yl)-4-(methylsulfonyl)pyridin-2-yl]prop anoate

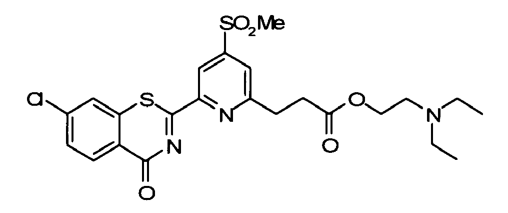

A mixture of 3-[6-(7-chloro-4-oxo-4H-1,3-benzothiazin-2-yl)-4-(methylsulfonyl)pyridin-2-yl]prop anoic acid (0.21 g, 0.5 mmol), N,N-diethylethanolamine (0.12 g, 1.0 mmol), HOBt H₂O (92 mg, 0.6 mmol), WSC (116 mg, 0.6 mmol), 4-dimethylaminopyridine (74 mg, 0.6 mmol) and DMF (3 ml) was stirred at room temperature for 18 hours. Water (20 ml) was added to the reaction mixture and the crystals formed were taken by filtration. The crystals were recrystallized from ethanol to give the title compound (0.13 g, 50%).
Melting point: 150.5-151.5°C
¹H-NMR (CDCl₃) δ: 1.02 (6H, t, J=7.2 Hz), 2.57 (4H, q, J=7.2 Hz), 2.71 (2H, t, J=6.3 Hz), 3.04 (2H, t, J=7.2 Hz), 3.16 (3H, s), 3.39 (2H, t, J=7.2 Hz), 4.20 (2H, t, J=6.3 Hz), 7.60-7.67 (2H, m), 7.98 (1H, s), 8.50 (1H, d, J=8.4 Hz), 8.80 (1H, s).

| Elemental analysis as C₂₃H₂₆N₃O₅S₂Cl | | | |
|---|---|---|---|
| Calcd.: | C, 52.71 ; | H, 5.00 ; | N, 8.02 |
| Found: | C, 52.48 ; | H, 4.91 ; | N, 7.97 |

### EXAMPLE 23

### 2-(Diethylamino)-2-oxoethyl 3-[6-(7-chloro-4-oxo-4H-1,3-benzothiazin-2-yl)-4-(methylsulfonyl)pyridin-2-yl]prop anoate

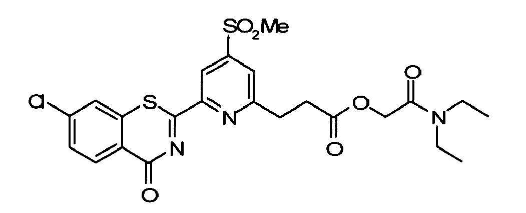

A mixture of 3-[6-(7-chloro-4-oxo-4H-1,3-benzothiazin-2-yl)-4-(methylsulfonyl)pyridin-2-yl]prop anoic acid (0.21 g, 0.5 mmol), N,N-diethyl-2-hydroxyacetamide (0.13 g, 1.0 mmol), HOBt H₂O (92 mg, 0.6 mmol), WSC (116 mg, 0.6 mmol), 4-dimethylaminopyridine (74 mg, 0.6 mmol) and DMF (3 ml) was stirred at room temperature for 18 hours. Water (20 ml) was added to the reaction mixture and the crystals formed were taken by filtration. The crystals were recrystallized from ethanol to give the title compound (0.21 g, 78%).
Melting point: 178.8-180.2°C
¹H-NMR (CDCl₃) δ: 1.11-1.24 (6H, m), 3.17 (3H, s), 3.17-3.25 (6H, m), 3.36-3.47 (2H, m), 4.77 (2H, s), 7.60-7.63 (1H, m),7.76 (1H, d, J=2.1 Hz), 8.01 (1H, s), 8.49 (1H, d, J=8.4 Hz), 8.80 (1H, s).

| Elemental analysis as C₂₃ H₂₄ N₃ O₆ S₂ C1.0.5 H₂O | | | |
|---|---|---|---|
| Calcd.: | C, 50.50 ; | H, 4.61 ; | N, 7.68 |
| Found: | C, 50.68 ; | H, 4.41 ; | N, 7.75 |

### EXAMPLE 24

### Sodium 3-[6-(7-chloro-4-oxo-4H-1,3-benzothiazin-2-yl)-4-(methylsulfonyl)pyridin-2-yl]prop anoate

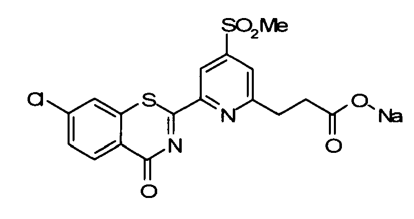

An acetone solution (10 ml) of sodium 2-ethylhexanoate (91 mg, 0.53 mmol) was added to an acetone solution (500 ml) of 3-[6-(7-chloro-4-oxo-4H-1,3-benzothiazin-2-yl)-4-(methylsulfonyl)pyridin-2-yl]prop anoic acid (0.21 g, 0.5 mmol) at room temperature and the mixture was allowed to stand for 18 hours. The crystals precipitated were taken by filtration to give the title compound (0.20 g, 90%) as crystals.
¹H-NMR (DMSO-d₆) δ: 2.49 (2H, t, J=7.5 Hz), 3.15 (2H, t, J=7.5 Hz), 3.41 (3H, s), 7.76-7.79 (1H, m), 8.15 (1H, s), 8.25 (1H, s), 8.34 (1H, d, J=8.4 Hz), 8.46 (1H, s).

### EXAMPLE 25

### Potassium 3-[6-(7-chloro-4-oxo-4H-1,3-benzothiazin-2-yl)-4-(methylsulfonyl)pyridin-2-yl]prop anoate

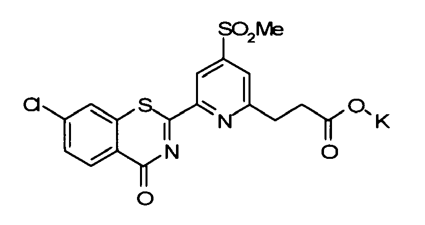

An acetone solution (10 ml) of potassium 2-ethyl hexanoate (116 mg, 0.55 mmol) was added to an acetone solution (500 ml) of 3-[6-(7-chloro-4-oxo-4H-1,3-benzothiazin-2-yl)-4-(methylsulfonyl)pyridin-2-yl]prop anoic acid (0.21 g, 0.5 mmol) at room temperature, and the mixture was allowed to stand for 60 hours. The crystals precipitated were taken by filtration to give the title compound (0.22 g, 95%).
¹H-NMR (DMSO-d₆) δ: 2.34 (2H, t, J=7.5 Hz), 3.11 (2H, t, J=7.5 Hz), 3.41 (3H, s), 7.78 (1H, dd, J=2.1 Hz, 8.4 Hz), 8.15 (1H, d, J=1.5Hz), 8.28 (1H, d, J=1.8 Hz), 8.35 (1H, d, J=8.4 Hz), 8.46 (1H, d, J=1.5 Hz).

| Elemental analysis as C₁₇H₁₂N₂O₅S₂ClK | | | |
|---|---|---|---|
| Calcd.: | C, 44.10 ; | H, 2.61 ; | N, 6.05 |
| Found: | C, 44.13 ; | H, 2.72 ; | N, 5.87 |

### EXAMPLE 26

### 3-[6-(7-chloro-4-oxo-4H-1,3-benzothiazin-2-yl)-4-(methylsulfonyl)pyridin-2-yl]prop anoic acid tromethamine salt

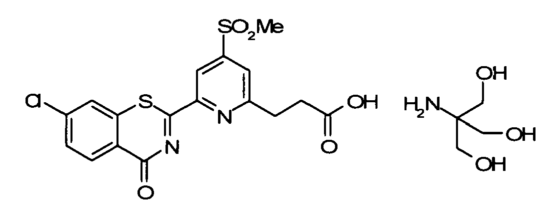

An acetone solution (500 ml) of 3-[6-(7-chloro-4-oxo-4H-1,3-benzothiazin-2-yl)-4-(methylsulfonyl)pyridin-2-yl]prop anoic acid (0.21 g, 0.5 mmol) was added to an acetone solution (1500 ml) of tromethamine (66.6 mg, 0.55 mmol) at room temperature, and the mixture was allowed to stand for 60 hours. The reaction mixture was concentrated under reduced pressure and the crystals precipitated were taken by filtration to give the title compound (0.18 g, 66%).
¹H-NMR (DMSO-d₆) δ: 2.77 (2H, t, J=7.5 Hz), 3.25 (2H, t, J=7.5 Hz), 3.43 (3H, s), 5.05 (12H, brs), 7.78 (1H, dd, J=2.1 Hz, 8.4 Hz), 8.20-8.22 (2H, m), 8.34 (1H, d, J=8.4 Hz), 8.49 (1H, s).

| Elemental analysis as C₂₁H₂₄N₃O₈S₂Cl·0.5 H₂O | | | |
|---|---|---|---|
| Calcd.: | C, 45.44 ; | H, 4.54 ; | N, 7.57 |
| Found: | C, 45.26 ; | H, 4.42 ; | N, 7.50 |

| PREPARATION EXAMPLE 1 | |
|---|---|
| Capsules | |
| (1) Compound obtained in EXAMPLE 8 | 30 mg |
| (2) Lactose | 60 mg |
| (3) Microcrystalline cellulose | 9 mg |
| (4) Magnesium stearate | 1 mg |
| 1 Capsule | 100 mg |

After mixing (1), (2) and (3) and a half of (4), the mixture is granulated. The remaining (4) is added to the granules and the resulting mixture is sealed in gelatin capsules.

| PREPARATION EXAMPLE 2 | |
|---|---|
| (1) Compound obtained in EXAMPLE 8 | 30 mg |
| (2) Lactose | 48 mg |
| (3) Corn starch | 18 mg |
| (4) Microcrystalline cellulose | 3.5 mg |
| (5) Magnesium stearate | 0.5 mg |
| 1 Tablet | 100 mg |

After mixing (1), (2), (3) and (4) and a half of (5), the mixture is granulated. The remaining (4) and (5) are added to the granules and the resulting mixture is compressed under pressure to make tablets.

| PREPARATION EXAMPLE 3 | |
|---|---|
| Capsules | |
| (1) Compound obtained in EXAMPLE 13 | 30 mg |
| (2) Lactose | 60 mg |
| (3) Microcrystalline cellulose | 9 mg |
| (4) Magnesium stearate | 1 mg |
| 1 Capsule | 100 mg |

After mixing (1), (2) and (3) and a half of (4), the mixture is granulated. The remaining (4) is added to the granules and the resulting mixture is sealed in gelatin capsules.

### EXPERIMENTAL EXAMPLE 1

### Experimental Example of ARE assay

### Assay for ARE-dependent transcriptional activation activity

Rat H9c2 cells were suspended in Dulbecco's Modified Eagle Medium (10% FBS, D-MEM) supplemented with 5% heat-inactivated fetal calf serum to 7 x 10⁴ cells/ml and a 18 ml aliquot was seeded on a Petri dish for cell culture, followed by incubation in 5% CO₂ at 37°C for about 16 hours. ARE (gagcttggaaatggcattgctaatggtgacaaagcaactttg; SEQ ID NO: 1) of rat glutathione S-transferase Ya subunit gene was incorporated into pGL3-Promoter Vector (manufactured by Promega Corp.) and the resulting luciferase reporter vector pGL3-ARE was transfected to the cells using FuGENE6 (Transfection reagent: manufactured by Roche), followed by additional incubation for about 7 hours. After completion of the incubation, the cells were recovered and suspended in 10% FBS-containing D-MEM to 1 x 10⁵ cells /ml. A 100 µl aliquot was seeded on each well of a 96-well plate, followed by incubation in 5% CO₂ at 37°C for about 17 hours. Next, the cells were washed with D-MEM to remove serum, and D-MEM containing a test compound (compound obtained in EXAMPLE 2, 8, 10, 12, 22 or 23) was added to the cells, followed by incubation in 5% CO₂ at 37°C for about 24 hours. After completion of the incubation, 80 µl each of Steady-Glo Reagent (manufactured by Promega Corp.) was added to each well. After allowing to stand at room temperature for 40 minutes, fluorescence levels by luciferase were determined using a WALLAC ARVO SX (manufactured by Perkin-Elmer). Taking the emitted luminescence of test compound-free group as 100%, the ARE-dependent transcriptional activation was shown in terms of the concentration of test compound, which caused to emit the luminescence of 200% (EC₂₀₀: µM). The results are shown in TABLE 1.

**TABLE 1**

| Test Compound | EC₂₀₀ (µM) |
|---|---|
| Compound of EXAMPLE 2 | 1.3 |
| Compound of EXAMPLE 8 | 0.94 |
| Compound of EXAMPLE 10 | 1.7 |
| Compound of EXAMPLE 12 | 1.6 |
| Compound of EXAMPLE 22 | 0.61 |
| Compound of EXAMPLE 23 | 0.6 |

### EXPERIMENTAL EXAMPLE 2

### Experimental Example of PPARα assay

### Assay for PAR-response element (PPRE)-dependent transcriptional activation activity

COS-1 cells were suspended in Dulbecco's Modified Eagle Medium (10% FBS, D-MEM) supplemented with 10% heat-inactivated fetal calf serum to 1 x 10⁵ cells/ml and a 25 ml aliquot was seeded on a75 cm² cell culture flask, followed by incubation in 5% CO₂ at 37°C for about 15 hours. PPARα expression vector pMCMV-neo-rPPARα and RXRα expression vector pMCMV-neo-rRXRα, in which human PPARα gene and human RXRα gene were inserted into pMCMV-neo (manufactured by Promega Corp.) respectively, and Luciferase reporter vector pGL3-PPRE4-Tk, in which four PPREs (aggacaaaggtca; SEQ ID NO: 2) were inserted into pGL3-Promoter Vector (manufactured by Promega Corp.) in tandem, were transfected to the cells using FuGENE6 (Transfection reagent: manufactured by Roche), and then incubation for about 24 hours. After completion of the incubation, the cells were recovered and suspended in 0.1% bovine serum albumin (Sigma)-containing D-MEM to 5 x 10⁴ cells /ml. A 100 µl aliquot was seeded on each well of a 96-well plate, followed by incubation in 5% CO₂ at 37°C for about 4 hours. Next, D-MEM containing a test compound (compound obtained in EXAMPLE 2, 8, 10 or 12) was added thereto, followed by incubation in 5% CO₂ at 37°C for about 42 hours. After completion of the incubation, a 80 µl aliquot was added to each well of Steady-Glo Reagent (manufactured by Promega Corp.). After allowing to stand for 30 minutes at room temperature, fluorescence levels by luciferase were determined using a WALLAC ARVO SX (manufactured by Perkin-Elmer). Taking the emitted luminescence of test compound-free group as 100%, the PPRE-dependent transcriptional activation was shown in terms of the concentration of test compound, which caused to emit the luminescence of 200% (EC₂₀₀: µM). The results are shown in TABLE 2.

**TABLE 2**

| Test Compound | EC₂₀₀ (µM) |
|---|---|
| Compound of EXAMPLE 2 | >100 |
| Compound of EXAMPLE 8 | >100 |
| Compound of EXAMPLE 10 | >100 |
| Compound of EXAMPLE 12 | >100 |

### EXPERIMENTAL EXAMPLE 3

### Test for binding to macrophage migration inhibitory factor (MIF)

### (1) Construction of human MIF protein expression vector

Using a sense strand (5'-ccatatgccgatgttcatcgtaaacac-3'; SEQ ID NO: 3) which coincided with the N terminus of human MIF containing the NdeI cleavage site at the 5' end and an antisense strand (5'-gaagaagctcttccgcaggcgaaggtggagttgttccag-3'; SEQ ID NO: 4) which coincided with the C terminus of human MIF containing the SapI cleavage site at the 5' end, the region encoding MIF was amplified from human complementary DNA (cDNA) library (GIBCO BRL, Inc.) by polymerase chain reaction (PCR). After the amplified MIFcDNA was cleaved with NdeI and SapI, the cleavage products were inserted between the NdeI and SapI sites of pCYB 1 (IMPACT I: One-Step Protein Purification System, New England BioLabs) to give pCYB1-hMIF. Next, after pCYB1-hMIF was cleaved with BglI, the cleaved site was blunt ended and then further cleaved with NdeI to recover human MIF and the intein-chitin binding domain fusion protein-encoding DNA fragment. Next, the recovered DNA fragment was inserted between the SapI and EcoRV sites of T7 promoter expression plasmid pET32b(+) (Novagen) to give human MIF-intein-chitin binding domain fusion protein expression plasmid pET32b-hMIF-Int-CBD. The MIFcDNA in the expression plasmid obtained was confirmed using a DNA Sequence System (Applied Biosystems, Inc.).

### (2) Preparation of human MIF Protein

After pET32b-hMIF-Int-CBD was transfected to Escherichia coli BL21 (DE3) (Novagen), the cells were inoculated on ampicillin-supplemented LB medium (1% tryptone, 0.5% yeast extract, 0.5% NaCl) (LBamp medium), followed by incubation while shaking at 37°C overnight. The cells were transferred to LBamp medium at a density of 1% and incubated while shaking at 37°C for about 2.5 hours. After 0.4 mM isopropyl-1-thio-β-D-galactopyranoside (IPTG) was added thereto, incubation was further continued at 15°C for about 24 hours to induce the expression of rat MIF-intein-chitin binding domain fusion protein. After completion of the incubation, Escherichia coli was recovered and suspended in a column buffer (20 mM Tris-HCl; pH 8.0, 500 mM NaCl, 0.1 mM EDTA) containing 1/10 volume of 0.1% Triton X-100, followed by ultrasonication. The cell lysate was centrifuged at 4°C and 12000 rpm for 30 minutes to recover the supernatant. The recovered supernatant was passed through a chitin bead column (New England BioLabs), which had been equilibrated with the column buffer containing 0.1% Triton X-100, to bind the MIF-intein-chitin binding domain fusion protein to the column. Thereafter, the column was washed with 0.1% Triton X-100-containing column buffer in a 10-fold volume of the column size and the column buffer in a 10-fold volume of the column size to remove non-specifically bound proteins and accompanied substances. Next, the buffer within the column was replaced by a column buffer containing 50 mM dithiothreitol. The column was allowed to stand at 4°C overnight, thereby to excise the MIF protein from the fusion protein, utilizing the protein splicing activity of intein. The excised MIF protein was eluted by the column buffer, followed by dialysis to 20 mM sodium phosphate buffer.

### (3) Binding of MIF to Compound of EXAMPLE 8

The binding of human MIF obtained in (2) above to the compound obtained in EXAMPLE 8 was analyzed using BIACORE 3000 (manufactured by Biacore). Human MIF was immobilized on a sensor chip CM5 (manufactured by Biacore). Then, phosphate buffered saline (PBS) containing the compound of EXAMPLE 8 was flown over the sensor chip and changes in the surface plasmon resonance signal were monitored as the binding of the compound to human MIF. In FIG 1, the ordinate designates the surface plasmon resonance signals (resonance units) and the abscissa designates time (seconds). The concentrations of the compound are shown by red: 0 µM, pink; 10 µM, green; 20 µM, light blue; 40 µM, dark blue; 60 µM and brown; 80 µM.

### INDUSTRIAL APPLICABILITY

In preferred embodiments, Compound (I) of the present invention is capable of binding to MIF, has excellent pharmacokinetics (oral absorption), excellent cytoprotective activities and cell death inhibitory activities, and is thus useful as pharmaceuticals having minimized side effects, cytoprotective agents, etc. In addition, the compound has the activities of increasing the expression of genes (e.g., genes for factors protecting cells from various stresses, etc.) under control of ARE, activities of upregulating (promoting) the production or activation of gene proteins (gene products) under control of ARE, etc. and is also useful as ARE activators. Therefore, Compound (I) of the present invention is useful as pharmaceuticals including agents for preventing/treating circulatory disorders, bone/joint disorders, infectious disorders, inflammatory disorders, renal disorders, central nervous disorders, cancer or diabetes mellitus, and antiaging agents, etc.

## Claims

1. A compound represented by formula (I): 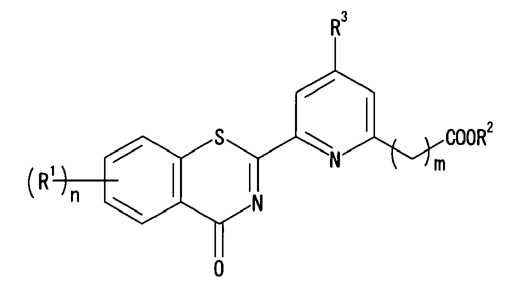 wherein R¹ independently represents a halogen atom, hydroxy, nitro, an optionally halogenated alkyl, alkenyl or alkynyl, an optionally substituted alkoxy, an acyl, or an optionally substituted amino; R² represents hydrogen atom or an optionally substituted hydrocarbon group; R³ represents an electron-withdrawing group; n represents an integer of 0 to 2; and m represents an integer of 1 to 6; or a salt thereof.

2. The compound according to claim 1 or a salt thereof, wherein R¹ is a halogen atom.

3. The compound according to claim 1 or a salt thereof, wherein R² is hydrogen atom or a C₁₋₆ alkyl group.

4. The compound according to claim 1 or a salt thereof, wherein the electron-withdrawing group is a halogen atom, cyano group, nitro group, a halogenomethyl group, a group represented by formula -S(O)ₚ-R⁴ (wherein p represents 1 or 2 and R⁴ represents hydrogen atom or an optionally substituted hydrocarbon group), a group represented by formula -COOR⁵ (R⁵ represents hydrogen atom or an optionally substituted hydrocarbon group), a group represented by formula -CONR⁶R⁷ (wherein R⁶ and R⁷ may be the same or different and independently represent hydrogen atom or an optionally substituted hydrocarbon group), a group represented by formula -SO₂NH-R⁸ (wherein R⁸ represents hydrogen atom or an optionally substituted hydrocarbon group), or a group represented by formula -CO-R⁹ (wherein R⁹ represents hydrogen atom or an optionally substituted hydrocarbon group).

5. The compound according to claim 1 or a salt thereof, wherein said compound or a salt thereof is selected from the group consisting of
3-[6-(4-oxo-4H-1,3-benzothiazin-2-yl)-4-(methylsulfonyl)pyridin-2-yl]propanoic acid;
3-[6-(7-chloro-4-oxo-4H-1,3-benzothiazin-2-yl)-4-(methylsulfonyl)pyridin-2-yl]prop anoic acid,
3-[6-(7-fluoro-4-oxo-4H-1,3-benzothiazin-2-yl)-4-(methylsulfonyl)pyridin-2-yl]propa noic acid;
3-[6-(7-methyl-4-oxo-4H-1,3-benzothiazin-2-yl)-4-(methylsulfonyl)pyridin-2-yl]prop anoic acid; 2-(diethylamino)ethyl
3-[6-(7-chloro-4-oxo-4H-1,3-benzothiazin-2-yl)-4-(methylsulfonyl)pyridin-2-yl]prop anoate; and 2-(diethylamino)-2-oxoethyl
3-[6-(7-chloro-4-oxo-4H-1,3-benzothiazin-2-yl)-4-(methylsulfonyl)pyridin-2-yl]prop anoate.

6. A macrophage migration inhibitory factor (MIF)-binding agent comprising the compound according to claim 1 or a salt thereof, or a prodrug thereof.

7. An antioxidant response element activator comprising the compound according to claim 1 or a salt thereof, or a prodrug thereof.

8. A cytoprotective agent comprising the compound according to claim 1 or a salt thereof, or a prodrug thereof.

9. A medicament comprising the compound according to claim 1 or a salt thereof, or a prodrug thereof.

10. The medicament according to claim 9, which is an agent for preventing/treating disorders caused by oxidative stress.

11. The medicament according to claim 9, which is an agent for preventing/treating circulatory disorders, bone/joint disorders, infectious disorders, inflammatory disorders, renal disorders, central nervous disorders, cancer or diabetes mellitus.

12. A method for preventing/treating circulatory disorders, bone/joint disorders, infectious disorders, inflammatory disorders, renal disorders, central nervous disorders, cancer or diabetes mellitus, which comprises administering to a mammal an effective dose of the compound according to claim 1 or a salt thereof, or a prodrug thereof.

13. Use of the compound according to claim 1 or a salt thereof, or a prodrug thereof, to manufacture an agent for preventing/treating circulatory disorders, bone/joint disorders, infectious disorders, inflammatory disorders, renal disorders, central nervous disorders, cancer or diabetes mellitus.
